# EUROPEAN PATENT APPLICATION

(11) **EP 2 080 772 A2**
(43) Date of publication of application: **22.07.2009**
(21) Application number: 09005494.1
(22) Date of filing: 21.11.2006
(51) Int. Cl.: C07K 14/72, C07K 17/00, C07K 19/00, C12N 15/11, G01N 33/78, A61K 39/00

(54) **Recombinant polypeptides and methods for detecting and/or quantifying autoantibodies against TSH receptor**

(30) Priority: 21.11.2005 US 738021 P
(62) Divisional of application: 06820995.6
(71) Applicant: Dr. Fenning BioMed GmbH, 79199 Kirchzarten (DE); MicroMol GmbH, 76199 Karlsruhe (DE)
(72) Inventor: The designation of the inventor has not yet been filed
(74) Representative: Maucher, Wolfgang

(57) **Abstract**

The present invention relates to unglycosylated isolated and purified recombinant polypeptides and novel epitopes of unglycosylated TSH receptor partial sequences able to bind to autoantibodies produced in response to an autoimmune disease associated with an immune reaction to a TSH-receptor. Also disclosed are methods of detecting and/or quantifying such autoantibodies using the isolated and purified recombinant polypeptides or novel epitopes and respective kits.

## Description

### The field of the invention

The present invention relates to unglycosylated isolated and purified recombinant polypeptides comprising a fusion protein able to bind to autoantibodies produced in response to an autoimmune disease associated with an immune reaction to a TSH-receptor (TSHR). Furthermore, it relates to methods of detecting and/or quantifying such autoantibodies using the isolated and purified recombinant polypeptides and to respective kits.

### Background of the invention

Thyroid diseases are the most common autoimmune diseases in humans and with a wide range of spectrum from Graves' disease (GD), Graves' ophthalmopathy (GO), Hashimoto's thyroiditis and idiopathic myxedema. Among them Graves' disease leads to increased of thyroid function, and clinical hyperthyroidism. This organ-specific autoimmune disease has an incidence of ∼4 in 10 000 people per year. Generally accepted mechanism of Graves' disease is, that the immune system produces autoantibodies directly against thyroid stimulating hormone (TSH) receptor and these kinds autoantibodies can mimic the function of natural produced thyroid stimulating hormone (TSH).

The established methods to detect TSH receptor autoantibodies (TRAbs) include biological assays measuring the functional effect (stimulatory or inhibitory of TRAbs on the TSHR signalling pathway), and *in vitro* assays detecting the inhibition of TSH binding to isolated or recombinanted TSH receptor.

### Cell line system:

A Chinese hamster ovary cell line is transfected with TSH receptor, which is expressed in the cell membrane. After patient serum is added to the cell line, the binding of TSHR autoantibodies to relevant epitopes of TSH receptor in the cell membrane is detected by stimulation or inhibition of new synthesized cyclic AMP upon to the treating of patient serum.

### Coating tube technology:

Coating tube technology developed by BRAHMS AG. The binding of TRAbs in the patient serum to TSHR coated on the tube is indirectly measured by inhibiting the interaction of radioactive labeled bovine TSH, so called TSH inhibitory immunoglobulin (TBII). TBII assay does not distinguish between thyroid stimulating antibodies (TSAbs) and thyroid blocking antibodies (TBAbs).

### Radio-Immuno-Assay (RIA):

Affinity purified TSHR from human or animal is labeled by radioactivity. After addition of patient serum, the autoantibodies in patient serum bind to TSHR forming an Antigen-Antibody complex. The complex is separated by immunoprecipitation, and the radioactivity is measured to evaluate the amount of autoantibodies in patient serum.

There are discrepancies among the various approaches, however, due to the heterogeneous nature of TRAbs. Recently monoclonal thyroid stimulating antibodies were identified for inhibition of hormone stimulation (Chae, C.B., et al., 2001, J Clin Endocrinol Metab 86(7):3311-3318. But the application is very limited, while the specificity and affinity of monoclonal antibodies is unknown and only two monoclonal cell lines were applied. The low sensitivity and the inability to distinguish the specificity of autoantibodies in current methods result in the contradictory of clinic study (DeGroot, L.J., 1990, J Clin Endocronol Metab 83 (11):3777-3785).

The use of different fragments of the human thyroid hormone receptor for the detection of autoantibodies produced in response to an autoimmune disease associated with an immune reaction to a TSH-receptor (anti-TSH receptor autoantibodies) has been described in EP-A 1078986. The different fragments were expressed in insect cells using a baculovirus signal sequence. It is disadvantageous, however, to use insect cells for the production of a glycosylated human protein. Proteins synthesized by insect cells may differ in their average carbohydrate composition as well as in their glycosylation pattern from natural occurring glycosylated human proteins. Consequently, the preparation of TSH receptor fragments using the baculovirus signal sequence and their expression in insect cells might lead to TSH receptor fragments differing in their carbohydrate composition and pattern from those expressed in the physiological situation by human cells.
The use of different fragments of the human thyroid hormone receptor for the detection of anti-TSH receptor autoantibodies has been described as well in WO 03/018632, wherein the fragments have been expressed in CHO cells. Proteins synthesized by CHO cells may also differ in their average carbohydrate composition from natural occurring glycosylated human proteins, however. Consequently, the TSH receptor proteins expressed by CHO cells may differ in their glycosylation pattern from natural occurring human TSH receptor and may therefore have binding properties different from natural occurring human TSH receptor in an assay for detection of anti-TSH receptor autoantibodies. Because of the low expression rate, the slow proliferation rate and the need of complex cultivation media supplemented with mammalian growth factors, production of TSH receptor in CHO cells is time consuming and expensive.
TSH receptor fragments of the extracellular domain of human TSHR containing amino acids 20-414 have been expressed as GST fusion protein in unglycosylated form in E. coli as described by Huang et al., 1995, Endocrinology 136(2): 588-593. The purified recombinant receptor preparations fail to show any binding to autoantibodies from patients with Graves' disease, however, indicating that the correct folding of the TSH receptor fragments maybe important for the binding of autoantibodies. This finding was underlined by Bobovnikova et al., 1997, J. Mol. Endocrinology 8 (2): 137-144, who obtained a soluble biologically active human TSH receptor fragment containing amino acids 21-415 by the expression of a GST fusion protein in a thioredoxin reductase mutant strain of E. coli which allows correct formation of disulfide bonds in its cytoplasm. The binding affinity of the TSH receptor fragments produced in the thioredoxin reductase mutant strain to labeled TSH was quite low, however, indicating that the properties of the obtained TSH receptor fragments differ considerably from natural occurring human TSH receptor.

The object of the present invention is to provide novel TSH receptor fragments for the detection of autoantibodies produced in response to an autoimmune disease associated with an immune reaction to a TSH-receptor, which have a high sensivity and low cross-reactivity and which can be used in a method for quantitative and qualitative detection of such autoantibodies.

### Summary of the invention

This object has been achieved with new unglycosylated isolated and purified recombinant polypeptides comprising a fusion protein able to bind to autoantibodies produced in response to an autoimmune disease associated with an immune reaction to a TSH-receptor, with unglycosylated TSH receptor partial sequences thereof and with novel epitopes of the unglycosylated TSH receptor partial sequences, and with novel methods for the detection and/or quantification of such autoantibodies using the new unglycosylated isolated and purified recombinant polypeptides, the unglycosylated TSH receptor partial sequences and/or the novel epitopes of the unglycosylated TSH receptor partial sequences.
Surprisingly, it has been found that unglycosylated isolated and purified recombinant polypeptides comprising a fusion protein, wherein one or more TSH receptor partial sequence is fused to a polypeptide, expressed in a prokaryotic host with no mutation allowing formation of disulfide bonds in the cytoplasm of the prokaryotic cells, have a high binding activity and a high binding specificity to autoantibodies against human TSHR and do show a low cross reactivity. These findings are contrary to the expectations with regard to the art discussed supra. Thus, the novel unglycosylated isolated and purified recombinant polypeptides of the present invention facilitate the detection, isolation and identification of novel autoantibodies produced in response to an autoimmune disease associated with an immune reaction to a TSH-receptor.

The above mentioned object and other objects as will be apparent from the following description have been achieved by providing new unglycosylated isolated and purified recombinant polypeptides comprising a fusion protein able to bind to autoantibodies produced in response to an autoimmune disease associated with an immune reaction to a TSH-receptor. Also provided are: unglycosylated TSH receptor partial sequences, an isolated and purified nucleic acid sequence encoding the fusion protein comprised by the unglycosylated isolated and purified recombinant polypeptides and novel epitopes of the unglycosylated TSH receptor partial sequences; a vector comprising at least one copy of said isolated and purified nucleic acid sequence and a prokaryotic host cell transformed with said isolated and purified nucleic acid sequence and/or said vector; methods of detecting and/or quantifying autoantibodies produced in response to an autoimmune disease associated with an immune reaction to a TSH-receptor in a biological fluid of a subject using the new unglycosylated isolated and purified recombinant polypeptides and/or the unglycosylated TSH receptor partial sequences and/or the novel epitopes of the unglycosylated TSH receptor partial sequences; novel autoantibodies produced in response to an autoimmune disease associated with an immune reaction to a TSH-receptor; a respective kit for detecting and/or quantifying autoantibodies produced in response to an autoimmune disease associated with an immune reaction to a TSH-receptor; use of the kit for the diagnosis of an autoimmune disease associated with an immune reaction to a TSH receptor; a pharmaceutical composition; the use of said unglycosylated isolated and purified recombinant polypeptides and/or said unglycosylated TSH receptor partial sequences and/or said novel epitopes of the unglycosylated TSH receptor partial sequences and/or said pharmaceutical composition for the preparation of a medicament for the therapeutic treatment or prevention of autoimmune disease associated with an immune reaction to a TSH receptor as well as a method of treating autoimmune disease associated with an immune reaction to a TSH receptor in a subject; further provided is a method for identifying a binding partner for a TSH receptor and a binding partner for a TSH receptor obtainable by said method.

Other objects and advantages will become apparent to those skilled in the art from a review of the ensuing detailed description, which proceeds with reference to the following illustrative drawings, and the attendant claims.

### Brief description of the figures

**Figure 1** shows the analysis of the GST-TSHR-fusion proteins by SDS-PAGE and Coomassie stain. From left to the right: TSHR-ED, TSHR-21 0, TSHR-31 0, blank, Protein Standard.
**Figure 2** shows the analysis of the GST-TSHR-fusion proteins by Western Blotting. From left to the right: TSHR-ED, TSHR-210, TSHR-310.

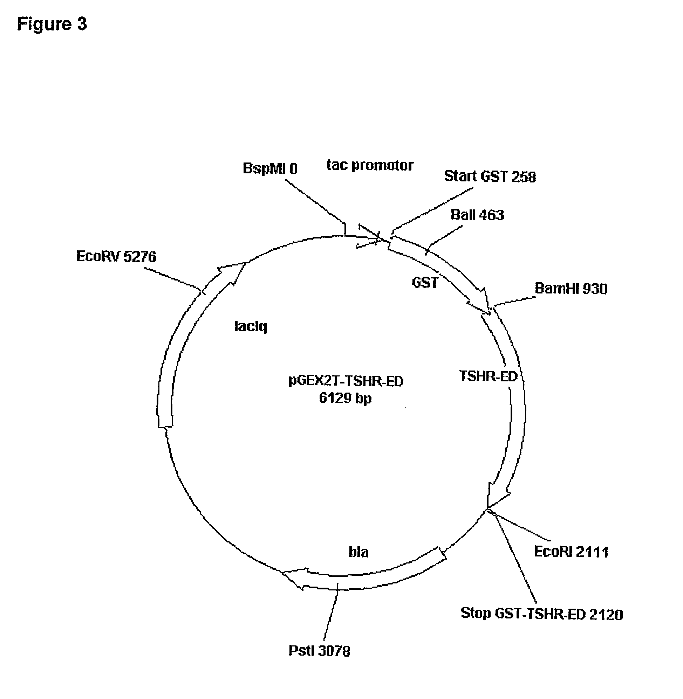

**Figure 3** shows plasmid pGEX2T-TSHR-ED containing the TSHR-ED fusion protein (TSHR-ED, GST) controlled by a tac promoter.
**Figure 4** shows the specific binding of the GST-TSHR-fusion proteins to TSH.
**Figure 5** shows the binding activity of GST-TSHR-fusion proteins to mouse anti-TSHR antibodies in microtiter plate, GST serves as negative control.
**Figure 6** shows the detection of anti-TSHR autoantibodies in commercial available pooled sera L1 and L2.
**Figure 7** shows the detection of anti-TSHR autoantibodies in patient sera and in commercial available pooled sera L1 and L2 by immobilized GST-TSHR-fusion proteins on the plastic wells of micotiter plates.
**Figure 8** shows a comparison of the signal responses of 90/672 (International Standard), L1 (commercial anti-TSHR positive sera) and H07 (patient serum) on GST, TSHR-ED and TSHR-310 coated plates.
**Figure 9** shows the detection of anti-TSHR autoantibodies isoforms in patient sera.
**Figure 10** shows the dose-dependent reaction of a mixture of GST-TSHR-fusion proteins (TSHR-ED and TSHR-310) to anti-TSHR autoantibodies in commercial available serum L2.

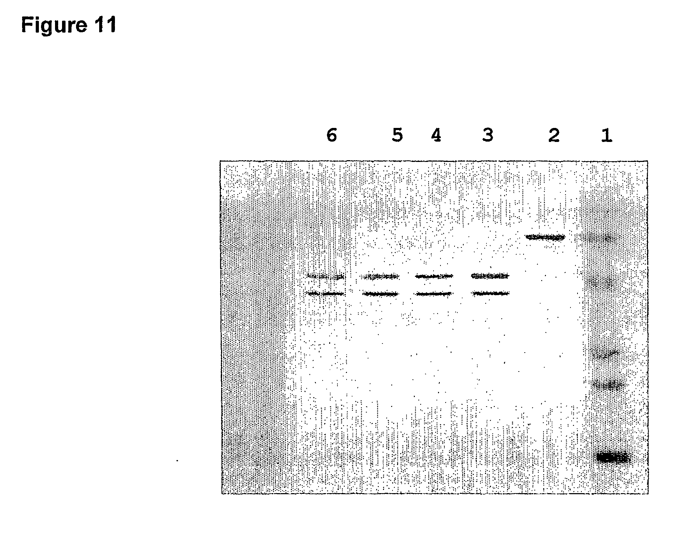

**Figure 11** shows cleavage of the GST-TSHR-fusion protein TSHR-ED with Thrombin. TSHR-ED and fragments thereof were visualized with anti-TSHR mAb 2C11 and secondary reagents. From right to left: Prestained Marker: 200, 119, 66, 43, 20, 14,3 kD; GST-TSHR-ED, without Thrombin; GST-TSHR-ED, with 0.1 U Thrombin; GST-TSHR-ED, with 0.5 U Thrombin; GST-TSHR-ED, with 1U Thrombin; GST-TSHR-ED, with 2 U Thrombin.
**Fig. 12** A. and B. show the binding activity of GST-tagged TSHR-ED protein either coated directly (white bars) or coated through glutathione (black bars) to IgG (A.) and IGM (B.) anti-TSHR autoantibodies in patient sera L2, L1, M4 and M21.
**Fig. 13** A. and B. show the binding activity of denatured and not denatures GST-TSHR-ED to IgG (A.) and IgM (B.). anti-TSHR autoantibodies in patient sera L2, L1, M4 and M21.
**Fig. 14** shows the reactivity of an individual serum sample (Serum M23) towards different peptides of the human TSHR.
**Figure 15** shows analysis of 38 patient's sera with the PepScan method. The X-Axis indicates the individual peptide, the Y-axis the frequency of the reacting sera.
**Figure 16** shows a schematic diagram of the extracellular Domain of the human TSRH with the identified hotspots (1-8). Unter the hotspot number the position of the identified amino acid domain (AA) as well as the number of reacting sera (in brackets) ae indicated.
**Figure 17** shows a detailed description and peptide sequences of the identified hotspots.
**Figure 18** shows the reactivity of Serum M460 towards TSHR specific peptides. On the very right side the reactivity against the positive control, the recombinant TSHR-ED (ED), is indicated.
**Figure 19** shows comparison binding activity of Serum 23 and Serum 460 with Recombinant Proteins (GST-TSHR-ED as well as negative control GST).
**Figure 20** shows core epitopes of the autoreactive hotspots. Sequences in square brackets represent the minimal epitope sequence.

### Detailed description of the invention

As used herein, the following definitions are supplied in order to facilitate the understanding of the present invention. As used herein, the terms "polypeptide", "peptide", "protein", "polypeptidic" and "peptidic" are used interchangeably to designate a series of amino acid sequence connected to the other by peptide bonds between the alpha-amino and carboxy groups of adjacent sequence.

As used herein, the term "fusion protein," refers to a composite protein, i.e., a single contiguous amino acid sequence, made up of two (or more) distinct, heterologous polypeptides which are not normally fused together in a single amino acid sequence. Thus, a fusion protein may include a single amino acid sequence that contains two (or more) distinct amino acid sequences, provided that these sequences are not normally found together in the same configuration in a single amino acid sequence found in nature.

"Unglycosylated TSH receptor partial sequence", "unglycosylated fusion protein" and "unglycosylated isolated and purified recombinant polypeptide" refer to polypeptide sequences not having formed a linkage with a glycosyl group.

"Fragments of amino acids sequences" or "fragments thereof' refer to an amino acid sequence that entirely is the same as part but not all of the amino acid sequence of the respective sequence of the polypeptide from which they derive. Usually, these sequences share at least 20% amino acids in length with the respective sequence of the polypeptide from which they derive. Preferably, these sequences share at least 50%, more preferably more than 80%, in particular more than 90% amino acids, more particular more than 95% amino acids in length with the respective sequence of polypeptide from which they derive. These sequences can be used as long as they exhibit the same properties, i. e. the biological function and activity of being able to bind to autoantibodies produced in response to an autoimmune disease associated with an immune reaction to a TSH-receptor, as the respective sequence of the polypeptide from which they derive.

The terms "variants of amino acid sequence" or "variants thereof" refer to polypeptides having amino acid sequences that differ to some extent from a reference native sequence polypeptide. Amino acid sequence variants will possess at least about 80% homology with the native sequence polypeptide. Preferably, they will be at least about 90%, in particular at least about 95% homologous, with such native sequence polypeptide. The amino acid sequence variants possess substitutions, deletions, and/or insertions at certain positions within the amino acid sequence of the native amino acid sequence. Usually, these amino acid sequences vary from the native sequence by amino acid substitutions, preferably conservative amino acid substitutions, whereby one or more amino acids are substituted by another with same characteristics. Conservative amino acid substitutions are herein defined as exchanges for example within one of the following five groups:
I. Small aliphatic, nonpolar or slightly polar sequence: Ala, Ser, Thr, Pro, Gly
II. Polar, positively charged sequence: His, Arg, Lys
III. Polar, negatively charged sequence: and their amides: Asp, Asn, Glu, Gln
IV. Large, aromatic sequence: Phe, Tyr, Trp
V. Large, aliphatic, nonpolar sequence: Met, Leu, Ile, Val, Cys.
   "Variants of amino acid sequence" or "variants" can be used as long as they exhibit the same properties, i. e. the biological function and activity of being able to bind to autoantibodies produced in response to an autoimmune disease associated with an immune reaction to a TSH-receptor, as the respective sequence of the polypeptide from which they derive. "Homology" is defined as the percentage of sequence in the amino acid sequence variant that are identical after aligning the sequences and introducing gaps, if necessary, to achieve the maximum percent homology. Methods and computer programs for the alignment are well known in the art.

A "vector" a "vector expressible in a host" or "expression vector" is a polynucleic acid construct, generated recombinantly or synthetically, with a series of specified polynucleic acid elements that permit transcription of a particular nucleic acid sequence in a host cell. Typically, this vector includes a transcriptional unit comprising a particular nucleic acid sequence to be transcribed operably linked to a promoter. A vector expressible in a host can be e. g. an autonomously or self-replicating plasmid, a cosmid or a phage.

The terms "host", "host cell" and "recombinant host cell" are used interchangeably herein to indicate a prokaryotic cell into which one or more vectors or isolated and purified nucleic acid sequences of the invention have been introduced. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

The term "isolated and purified nucleic acid sequence" refers to the state in which the nucleic acid sequence will be, in accordance with the present invention. The nucleic acid sequence will be free or substantially free of material with which it is naturally associated such as other nucleic acids with which it is found in their natural environment, or the environment in which it is prepared (e. g. cell culture) when such preparation is by recombinant technology practised in vitro or in vivo.

The term "cell transfected" or "cell transformed" or "transfected/transformed cell" means the cell into which an extracellular nucleic acid sequence has been introduced and thus harbors the extracellular nucleic acid sequence. The nucleic acid sequence might be introduced into the cell so that the nucleic acid is replicable either as a chromosomal integrant or as an extra chromosomal element.

"Antibody" refers to a class of plasma proteins produced by the B-cells of the immune system after stimulation by an antigen. Mammal (i.e. human) antibodies are immunoglobulins of the Ig G, M, A, E or D isotype. The term "antibody" as used for the purposes of this invention includes, but is not limited to, polyclonal antibodies, monoclonal antibodies, anti-idiotypic antibodies and auto-antibodies present in autoimmune diseases, as well as chimeric antibodies. The term antibody is used to mean whole antibodies and binding fragments thereof. Binding fragments include single chain fragments, Fv fragments and Fab fragments. The term Fab fragment is sometimes used in the art to mean the binding fragment resulting from papain cleavage of an intact antibody. The terms Fab' and F(ab')2 are sometimes used in the art to refer to binding fragments of intact antibodies generated by pepsin cleavage. In the context of the present invention, Fab is used to refer generically to double chain binding fragments of intact antibodies having at least substantially complete light and heavy chain variable domains sufficient for antigen-specific bindings, and parts of the light and heavy chain constant regions sufficient to maintain association of the light and heavy chains. Further encompassed are chimeric antibodies which are antibodies whose light and heavy chain genes have been constructed, typically by genetic engineering, from immunoglobulin gene segments (e. g., segments encoding the variable region and segments encoding the constant region), for example, belonging to different species.
"Autoantibodies produced in response to an autoimmune disease associated with an immune reaction to a TSH-receptor", "autoantibodies against TSHR" or "anti-TSHR antibodies" refer to antibodies directed against the TSH receptor which are capable to bind to the TSH receptor. Depending on the type of these autoantibodies, either inhibition of the formation and release of thyroid hormones T3 and T4 may occur, or, on the other hand, these thyroid hormones may be released in an uncontrolled manner because the anti-TSH receptor autoantibodies mimic the action of the TSH and stimulate the synthesis and release of thyroid hormones. Further autoantibodies may bind to the TSH receptor but do not stimulate thyroid hormone production and are described as having blocking activity.

Herein, a "subject" usually refers to a human or animal, preferably a human. More preferably a "subject" refers to a human or animal suspected of suffering from, susceptible to, having or recovering from autoimmune disease associated with an immune reaction to a TSH receptor.

Herein, an "epitope" means a portion or a site on an antigen, such as a polypeptide, with the ability or potential to elicit and combine with an antibody. Polypeptides often include more than one epitope. For the purpose of this disclosure, a polypeptide epitope will usually include at least 3 amino acis, preferably 5 to 50 amino acids, more preferably between about 5 to 20 amino acids, and most preferably between about 8 to 15 amino acids, referred herein as "core epitope", in the peptide. There is no critical upper limit to the length of the peptide, which could comprise nearly the full length of the polypeptide sequence. Epitopes can be either linear or conformational epitopes. A linear epitope is comprised of a single segment of a primary sequence of a polypeptide chain. Linear epitopes can be contiguous or overlapping. Conformational epitopes are comprised of amino acids brought together by folding of the polypeptide to form a tertiary structure and the amino acids are not necessarily adjacent to one another in the linear sequence.

In one aspect, the present invention provides an unglycosylated isolated and purified recombinant polypeptide comprising a fusion protein able to bind to autoantibodies produced in response to an autoimmune disease associated with an immune reaction to a TSH-receptor obtainable by the method comprising,
a) transforming a prokaryotic host with no mutation allowing formation of disulfide bonds in the cytoplasm of the prokaryotic host cells with a vector comprising an isolated and purified nucleic acid sequence encoding said fusion protein, wherein one or more TSH receptor partial sequences is fused to a polypeptide and wherein said TSH receptor partial sequences consist essentially of the extracellular domain of the TSH receptor, fragments thereof or variants thereof,
b) culturing said prokaryotic host cells whereby said fusion protein is expressed and accumulated in said prokaryotic host cells
c) isolating said prokaryotic host cells
d) lysing said prokaryotic host cells in the presence of a detergent
e) separating soluble components of the lysate from insoluble components
f) purifying said fusion protein from said soluble components of said lysate.

In a further aspect the present invention provides an unglycosylated isolated and purified recombinant polypeptide expressed in a prokaryotic host with no mutation allowing formation of disulfide bonds in the cytoplasm of the prokaryotic host cells, said unglycosylated isolated and purified recombinant polypeptide comprising a fusion protein able to bind to autoantibodies produced in response to an autoimmune disease associated with an immune reaction to a TSH-receptor, wherein one or more TSH receptor partial sequences is fused to a polypeptide consisting essentially of the glutathione S transferase (GST), fragments thereof or variants thereof, and wherein said TSH receptor partial sequence consists essentially of the extracellular domain of the TSH receptor, fragments thereof or variants thereof.

The TSH receptor partial sequence can be of human, pig, bovine, monkey or mouse origin. Preferably, the TSH receptor partial sequence of the present invention is of human origin. The amino acid sequence of the human TSH receptor is described e. g. in Frazier et al., Mol. Endocrinol. 4 (8), 1264-1276 (1990).

The polypeptide to be fused can be a polypeptide or fragments thereof or variants thereof known to the person skilled in the art. Polypeptides of prokaryotic or eukaryotic origin can be used. Among many others, typical commercially available polypeptides for the creation of fusion constructs e. g. glutathione S transferase (GST), beta-glucuronidase (GUS), green fluorescent protein (GFP) or prokaryotic binding proteins such as Maltose Binding protein (MBP) and Cellulose Binding Domain (CBD) can be used. As well open reading frame (orf) sequences or any other sequence of interest which may be desired or used e.g. in various recombinant techniques including sequences for use in homologous recombination (e. g. gene targeting) can be used. As well poly amino acid sequences such as poly histidine linker, T7-tag, myc-tag, Flag-tag or V5-tag can be used as polypeptide to be fused.

Usually, one TSH receptor partial sequence is fused to one polypeptide. The TSH receptor partial sequences of the present invention might be fused to the polypeptide at the N-terminus or the C-terminus of the polypeptide, preferably it is fused to the C-terminus of the polypeptide. The TSH receptor partial sequences can be directly fused to the polypeptide at the N-terminus or at the C-terminus of the polypeptide sequence, i. e. the last amino acid of the respective sequence is followed by the first amino acid of the other respective sequence or, preferably, additional amino acids are situated between both sequences as long as the biological function and activity of the fusion protein of being able to bind to autoantibodies produced in response to an autoimmune disease associated with an immune reaction to a TSH-receptor is maintained. In case the fusion protein contains additional amino acids between the last amino acid of the polypeptide and the first amino acid of the TSH receptor partial sequence, the number of these additional amino acids is usually limited to a maximum of four amino acids, preferably to a maximum of two amino acids, more preferably exactly two amino acids, particularly preferred two amino acids in the following order from the C-terminus of the polypeptide: glycine followed by serine.

The preferred polypeptide to be fused to the TSH receptor partial sequences of the present invention consists essentially of glutathione S transferase (GST), beta-glucuronidase (GUS), green fluorescent protein (GFP) or prokaryotic binding proteins such as Maltose Binding protein (MBP) or Cellulose Binding Domain (CBD), a fragment thereof or a variant thereof. More preferably, the polypeptide to be fused with the TSH receptor partial sequences of the present invention consists essentially of glutathione S transferase (GST), a fragment thereof or a variant thereof.
Usually, the fusion protein contains at the C-terminus of the TSH receptor partial sequence between one and ten, preferably between four and eight additional amino acids as long as the biological function and activity of the fusion protein of being able to bind to autoantibodies produced in response to an autoimmune disease associated with an immune reaction to a TSH-receptor is maintained. More preferably, the fusion protein contains at the C-terminus of the TSH receptor partial sequence exactly six additional amino acids. Most preferred is a fusion protein which contains six amino acids in the following order from the C-terminus of the TSH receptor partial sequence: glutamic acid followed by phenylalanine, followed by isoleucine, followed by valine, followed by threonine, and followed by aspartic acid.

"TSH receptor partial sequence" refers to an amino acid sequence that entirely is the same as the extracellular domain of the TSH receptor, fragments thereof or variants thereof. Usually, the extracellular domain of the TSH receptor, fragments thereof or variants thereof consists essentially of the amino acid sequence 20-418 from the N-terminus of a TSH. Preferably, the TSH receptor partial sequence consists essentially of one of the following amino acid sequences, fragments thereof or variants thereof:
amino acid sequence 22 to 415 from the N-terminus of a TSH receptor; amino acid sequence 210 to 415 from the N-terminus of a TSH receptor; and amino acid sequence 310 to 415 from the N-terminus of a TSH receptor.

More preferred is an unglycosylated isolated and purified recombinant polypeptide, wherein the TSH receptor partial sequence consists essentially of one of the following amino acid sequences, fragments thereof or variants thereof: SEQ ID No. 1, SEQ ID No. 2 and SEQ ID No. 3, in particular consisting essentially of SEQ ID No. 3.
Even more preferred is an unglycosylated isolated and purified recombinant polypeptide, wherein the TSH receptor partial sequence is selected from the group consisting of
a) an amino acid sequence comprising the amino acid sequence 22 to 415 from the N-terminus of a TSH receptor as shown in SEQ ID NO: 1;
b) an amino acid sequence which is at least 80 % identical to the amin acid sequence as defined in a);
c) an amino acid sequence comprising the amino acid sequence 210 to 415 from the N-terminus of a TSH receptor as shown in SEQ ID NO: 2;
d) an amino acid sequence which is at least 80 % identical to the amin acid sequence as defined in c);
e) an amino acid sequence comprising the amino acid sequence 310 to 415 from the N-terminus of a TSH receptor as shown in SEQ ID NO: 3;
f) an amino acid sequence which is at least 80 % identical to the amin acid sequence as defined in e);
   in particular an unglycosylated isolated and purified recombinant polypeptide,wherein the TSH receptor partial sequence is selected from the group consisting of the amino acids sequences as defined in e) and f).
   The amino acid sequences as defined in b), d) and f) comprise preferably an amino acid sequence which is at least 85 %, more preferably at least 90 %, most preferably at least 95 %, in particular at least 97 %, most particular at least 99 % identical to the amino acid sequence of reference as defined in a), c) and e) and preferably differs from the sequence of the reference by conservative amino acid substitutions.
   Most preferred is an unglycosylated isolated and purified recombinant polypeptide consisting essentially of one of the following amino acid sequences, fragments thereof or variants thereof: SEQ ID No. 4, SEQ ID No. 5 and SEQ ID No. 6, in particular consisting essentially of SEQ ID No. 6.
   Particularly preferred is an unglycosylated isolated and purified recombinant polypeptide selected from the group consisting of

a) an amino acid sequence comprising the amino acid sequence as shown in SEQ ID NO: 4;
b) an amino acid sequence which is at least 80 % identical to the amino acid sequence as defined in a);
c) an amino acid sequence comprising the amino acid sequence as shown in SEQ ID NO: 5;
d) an amino acid sequence which is at least 80 % identical to the amino acid sequence as defined in c);
e) an amino acid sequence comprising the amino acid sequence as shown in SEQ ID NO: 6;
f) an amino acid sequence which is at least 80 % identical to the amino acid sequence as defined in e),
   more particular an unglycosylated isolated and purified recombinant polypeptide selected from the group consisting of the amino acid sequences as defined in e) and f). The amino acid sequences as defined in b), d) and f) comprise preferably an amino acid sequence which is at least 85 %, more preferably at least 90 %, most preferably at least 95 %, in particular at least 97 %, most particular at least 99 % identical to the amino acid sequence of reference as defined in a), c) and e) and preferably differs from the sequence of the reference by conservative amino acid substitutions
   Even more particulalry preferred is an unglycosylated isolated and purified recombinant polypeptide consisting essentially of one of the following amino acid sequences: SEQ ID No. 4, SEQ ID No. 5 and SEQ ID No. 6, in particular consisting of SEQ ID No. 6. More particularly preferred is an unglycosylated isolated and purified recombinant polypeptide consisting of one of the following amino acid sequences: SEQ ID No. 4, SEQ ID No. 5 and SEQ ID No. 6, in particular consisting of SEQ ID No. 6.

Surprisingly, it has been found that the unglycosylated isolated and purified recombinant polypeptide of the present invention is able to bind to their physiological ligand TSH (thyroid-stimulating hormone) as well as to monoclonal anti-TSHR antibodies such as 2C11 and 4C1, both commercially available from Serotec GmbH, and to TSHR autoantibodies contained in 90/672, an international standard serum of thyroid stimulating antibodies (prepared from single patient) obtainable from the national institute for biological standards and control (NIBSC).

The unglycosylated isolated and purified recombinant polypeptide of the present invention might be digested or cleaved in order to obtain the respective TSH receptor partial sequence without the fused polypeptide.
Thus, a further embodiment of the present invention is an unglycosylated TSH receptor partial sequence able to bind to autoantibodies produced in response to an autoimmune disease associated with an immune reaction to a TSH-receptor, said unglycosylated TSH receptor partial sequence consisting essentially of the extracellular domain of the TSH receptor, fragments thereof or variants thereof obtainable by the method comprising,
a) transforming a prokaryotic host with no mutation allowing formation of disulfide bonds in the cytoplasm of the prokaryotic host cells with a vector comprising an isolated and purified nucleic acid sequence encoding a fusion protein able to bind to autoantibodies produced in response to an autoimmune disease associated with an immune reaction to a TSH-receptor, wherein one or more TSH receptor partial sequences is fused to a polypeptide and wherein said TSH receptor partial sequences consist essentially of the extracellular domain of the TSH receptor, fragments thereof or variants thereof,
b) culturing said prokaryotic host cells whereby said fusion protein is expressed and accumulated in said prokaryotic host cells
c) isolating said prokaryotic host cells
d) lysing said prokaryotic host cells in the presence of a detergent
e) separating soluble components of the lysate from insoluble components
f) purifying said fusion protein from said soluble components of said lysate
g) digesting or cleaving said polypeptide fused to said TSH receptor partial sequence; and
h) recovering said TSH receptor partial sequence, fragments thereof or variants thereof.

As a preferred unglycosylated TSH receptor partial sequence the TSH receptor partial sequence fused to the polypeptide consists essentially of one of the following amino acid sequences, fragments thereof or variants thereof:
amino acid sequence 22 to 415 from the N-terminus of a TSH receptor; amino acid sequence 210 to 415 from the N-terminus of a TSH receptor; and amino acid sequence 310 to 415 from the N-terminus of a TSH receptor.
   More preferred is an unglycosylated TSH receptor partial sequence selected from the group consisting of
   a) an amino acid sequence comprising the amino acid sequence 22 to 415 from the N-terminus of a TSH receptor as shown in SEQ ID NO: 1;
   b) an amino acid sequence which is at least 80 % identical to the amin acid sequence as defined in a);
   c) an amino acid sequence comprising the amino acid sequence 210 to 415 from the N-terminus of a TSH receptor as shown in SEQ ID NO: 2;
   d) an amino acid sequence which is at least 80 % identical to the amin acid sequence as defined in c);
   e) an amino acid sequence comprising the amino acid sequence 310 to 415 from the N-terminus of a TSH receptor as shown in SEQ ID NO: 3;
   f) an amino acid sequence which is at least 80 % identical to the amin acid sequence as defined in e).
in particular an unglycosylated isolated and purified recombinant polypeptide,wherein the TSH receptor partial sequence is selected from the group consisting of the amino acids sequences as defined in e) and f).
   The amino acid sequences as defined in b), d) and f) comprise preferably an amino acid sequence which is at least 85 %, more preferably at least 90 %, most preferably at least 95 %, in particular at least 97 %, most particular at least 99 % identical to the amino acid sequence of reference as defined in a), c) and e) and preferably differs from the sequence of the reference by conservative amino acid substitutions. Most preferably, the TSH receptor partial sequence fused to the polypeptide consists essentially of one of the following amino acid sequences, fragments thereof or variants thereof: SEQ ID No. 1, SEQ ID No. 2 and SEQ ID No. 3, in particular it consists essentially of SEQ ID No. 3.

The method by which the unglycosylated isolated and purified recombinant polypeptide and the respective unglycosylated TSH receptor partial sequence are obtainable as well as the method which can be applied to obtain the unglycosylated isolated and purified recombinant polypeptide expressed in a prokaryotic host with no mutation allowing formation of disulfide bonds in the cytoplasm of the prokaryotic host cells of the present invention can be carried out by generating first an isolated and purified nucleic acid sequence encoding the TSH receptor partial sequence which can be fused to a nucleic acid sequence encoding the desired polypeptide. The obtained construct can be ligated into a vector which usually harbours an appropriate regulatory sequence and the vector can be used to transform a respective prokaryotic host according to standard procedures well known in the art. As cell culture system continuous or discontinuous culture culture such as batch culture or fed batch culture can be applied in culture tubes, shake flasks or bacterial fermentors. Host cells are usually cultured in conventional media as known in the art such as complex media like "nutrient yeast broth medium" or Luria Berthani (LB) medium. The preferred medium for carrying out the expression of the polypeptide is Luria Berthani (LB) medium.
The medium might be modified as appropriate e.g. by adding further ingredients such as buffers, salts, vitamins or amino acids. As well different media or combinations of media can be used during the culturing of the cells. Appropriate pH ranges are e. g. 6 - 8 preferably 7 -7.5, appropriate culture temperatures are between 10 and 40, preferably between 20 and 37°C. The host cells are cultured usually as long as it takes until the expressed product has accumulated, preferably between 1 hour and 5 days. The isolation of the cells can be performed by methods known in the art such as e.g. centrifugation, which is preferred. Lysing of the isolated cells in the presence of a detergent is usually performed by resuspending the cells in a detergent or preferably resuspending the cells first in a buffer like TRIS buffered saline (TBS) or phosphate bufferd saline (PBS) followed by a further resupension in a detergent. As detergent usually one or more ionic detergent like SDS (Sodium dodecyl sulphate) or one or more non-ionic detergent like Triton X-100, Nonidet P40, N-Octylglukosid can be used. Preferably one or more non-ionic detergents are used, more preferably one non-ionic detergent, most preferably Triton X-1 00 is used. The detergent of the present invention does not comprise urea. The detergent is usually contained in a buffer like TRIS buffered saline (TBS) or phosphate bufferd saline (PBS) which might contain additionally a reducing compound like dithiothreitol (DTT) or mercaptoethanol. The detergent is usually applied at a concentration between 0,1 and 2 %, preferably between 0,5 and 1,5%. The isolated cells can be lysed by methods known in the art such as e.g. sonification, addition of lysozyme, mechanical disruption with metal beads in a cell homogenizer, preferably by sonification. Preferably, the bacterial lysate is incubated, more preferably at low temperatures e. g. on ice for a time period between 1 and 60 min, preferably between 20 and 40 min to obtain maximum solubilization of the proteins.
Separation of soluble components of the lysate from insoluble components can be performed by methods known in the art. Usually, methods which do not affect the conformation of the fusion protein are used such as e.g. centrifugation which is preferred. The fusion protein is then purified from the soluble components of the lysate by protein purification procedures known in the art which may include differential precipitation, molecular sieve chromatography, ion-exchange chromatography, isoelectric focusing, gel electrophoresis, affinity chromatography, and immunoaffinity chromatography. These well known and routinely practiced methods are described in, e.g., Ausubel et al., 1994, Current protocols in molecular biology, John Wiley and Sons, and Wu et al. (eds.), Academic Press Inc., N.Y.; Immunochemical Methods In Cell And Molecular Biology. Usually, methods which do not affect the conformation of the fusion protein are used, like e. g. molecular sieve chromatography, ion-exchange chromatography, isoelectric focusing, gel electrophoresis, affinity chromatography, and immunoaffinity chromatography, with affinity chromatography preferred. In case GST is used as fused polypeptide, glutathione affinity chromatography such as glutathione sepharose chromatography is preferably used for purifying the fusion protein from the soluble components of the lysate. In case of the use of poly histidine linker as additional amino acids, the purification can be carried out using immobilised metal affinity chromatography like Ni-agarose. The purified fusion protein can be dialysed against a commonly known buffer like TBS and can be used dissolved in such a buffer for the method of detecting and/or quantifying autoantibodies of the present invention.

In case the purified fusion protein is further digested or cleaved to obtain the respective TSH receptor partial sequence, digestion or cleavage can be performed according to methods known by the skilled in the art, such as chemical digestion of the polypeptide fused or enzymatic cleavage between the polypeptide fused and the respective TSH receptor partial sequence by e. g. specific proteases like thrombin. The respective TSH receptor partial sequence can be recovered after digestion or cleavage by known methods such as gel electrophoresis or affinity chromatography. As can be seen from example 11, the TSH receptor partial sequence of a fusion protein of the present invention is cleaved by thrombin in two fragments of an apparent molecular weight of around 45 kDa and around 35 kDa indicating that an unglycosylated isolated and purified recombinant polypeptide of the present invention has at least one thrombin cleavage region within the TSH receptor partial sequence. The preferred fusion protein of the present invention which is cleaved by thrombin in two fragments of an apparent molecular weight of around 45 kDa and around 35 kDa indicating that an unglycosylated isolated and purified recombinant polypeptide of the present invention has at least one thrombin cleavage region within the TSH receptor partial sequence is the unglycosylated isolated and purified recombinant polypeptide, selected from the group consisting of a) an amino acid sequence comprising the amino acid sequence as shown in SEQ ID NO: 4;
b) an amino acid sequence which is at least 80 % identical to the amino acid sequence as defined in a).

Further provided by the present invention is an epitope of an unglycosylated TSH receptor partial sequence selected from the group consisting of
a) an epitope comprising at least 5 consecutive amino acids of the amino acid sequence selected from SEQ ID NOS: 10-27;
b) an epitope comprising an amino acid sequence selected from SEQ ID NOS: 10-27;
c) an epitope which is at least 60 % identical to the amino acid sequences defined in a) or b).

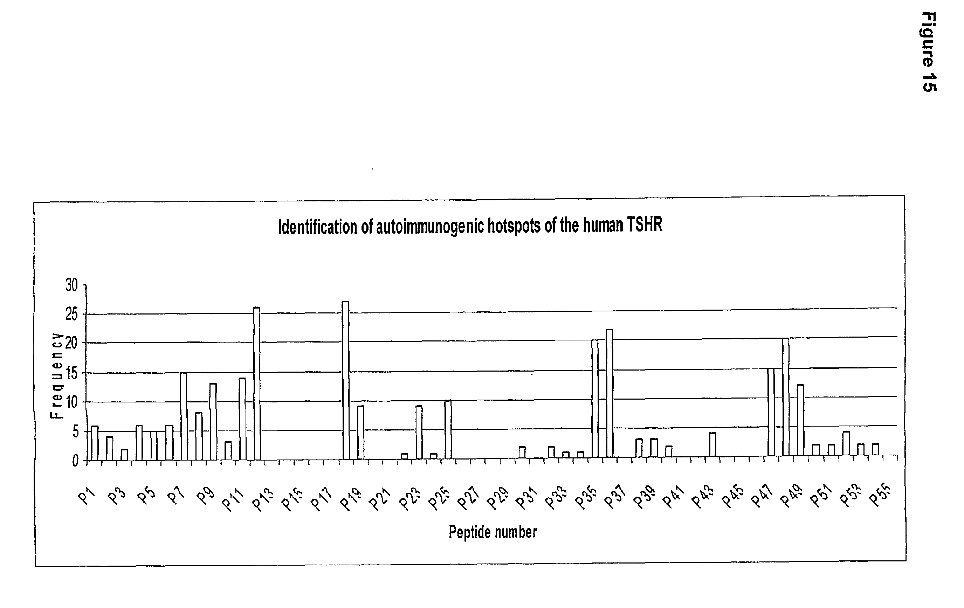

Epitopes of a) preferably comprise at least 8 consecutive amino acids of the amino acid partial sequence selected from SEQ ID NOS: 10-27.
Preferred epitopes selected from the group consisting of a)-c) show a reactivity with at least 15, preferably 20 of 38 patient's sera containing anti-TSHR autoantibodies as shown in Fig. 15.
More preferred epitopes of the present invention are epitopes of an unglycosylated TSH receptor partial sequence selected from the group consisting of
a) an epitope comprising at least 5 consecutive amino acids of the amino acid sequence selected from SEQ ID NOS: 12, 13, 16, 17, 20, 21, 26 and 27;
b) an epitope comprising at least 5 consecutive amino acids of the amino acid sequence selected from SEQ ID NOS: 12, 13, 16, 17, 20, 21, 26 and 27;
c) an epitope which is at least 60 % identical to the amino acid sequences defined in a) or b).
   Epitopes as defined in c) are preferably at least 70 %, more preferably at least 80 %, most preferably at least 90 %, in particular at least 95 % identical to the amino acid sequences as defined in (a) or (b)

Further provided is an isolated and purified nucleic acid sequence encoding the fusion protein comprised by the unglycosylated isolated and purified recombinant polypeptide of the present invention. The isolated and purified nucleic acid sequences encompassed by the present invention might be DNA or cDNA, RNA, or DNA/RNA hybrid. Usually, it is DNA or cDNA.
Preferably, the isolated and purified nucleic acid sequence encoding the fusion protein comprised by the unglycosylated isolated and purified recombinant polypeptide consists essentially of SEQ ID No. 7, SEQ ID No. 8 or SEQ ID No. 9, more preferably of SEQ ID No. 9.

Said purified and isolated nucleic acid sequence usually further comprises one or more regulatory sequences, as known in the art e.g. a promoter and/or an enhancer, polyadenylation sites and splice junctions usually employed for the expression of a protein or may optionally encode a selectable marker. Preferably, said purified and isolated nucleic acid sequence comprises a promoter which is operably linked to the sequence or the gene of interest. The nucleic acid sequences of this invention can be isolated according to standard PCR protocols and methods well known in the art. The present invention also includes nucleic acid sequences that vary from the reference sequence according to the degeneration of the genetic code and by conservative nucleic acid substitutions, whereby one or more nucleic acid are substituted by another with same characteristics.

The isolated and purified nucleic acid sequences of the present invention are usually located on a vector. As well encompassed by the present invention is therefore a vector comprising at least one copy of the isolated and purified nucleic acid sequence of the present invention.
The vector according to the invention is preferably an autonomously or self-replicating plasmid, a cosmid or a phage. A wide variety of host/vector combinations may be employed in expressing the nucleic acid sequences of this invention. Useful vectors, for example, may consist of segments of chromosomal, non-chromosomal and/or synthetic nucleic acid sequences. Suitable vectors include vectors with specific host range such as vectors specific for e. g. E. coli as well as vectors with broad host range such as vectors useful for gram-negative bacteria. "Low-copy", "medium-copy" as well as "high copy" plasmids can be used. Useful vectors for e. g. expression in E. coli are: pQE70, pQE60 und pQE-9 (QIAGEN,Inc.); pBluescript Vektoren, Phagescript Vektoren, pNH8A,pNH16a,pNH18A, pNH46A (Stratagene Cloning Systems, Inc.); ptrc99a, pKK223-3, pKK233-3, pDR540, pRIT5 (Pharmacia Bio-tech, Inc.), pGEX-2T (Amersham Biosciences), pET vectors like pET14b (Novagen) or derivates thereof. Further useful plasmids are well known to the person skilled in the art and are described e.g. in "Cloning Vectors" (Eds. Pouwels P. H. et al. Elsevier, Amsterdam-New York-Oxford, 1985).
Preferred vectors of the present inventions are autonomously or self-replicating plasmids, more preferred are vectors with specific host range such as vectors specific for e. g. E. coli. Most preferred are pGEX-2T or pET vectors like pET14b or derivates thereof, particularly preferred is pGEX-2T or derivates thereof. More particular preferred are pGEX2T-TSHR-ED, pGEX2T-TSHR-210, and pGEX2T-TSHR-310.

The isolated and purified recombinant polypeptide and the TSH receptor partial sequence of the present invention can be produced in various prokaryotic host cells with no mutation allowing formation of disulfide bonds in their cytoplasm and which can be transformed with the respective nucleic acid sequence. Thus, a further object of the present invention is a prokaryotic host cell with no mutation allowing formation of disulfide bonds in its cytoplasm transformed with the nucleic acid sequence and/or the vector of the present invention. The prokaryotic host cell of the present invention is normally not able to form disulfide bonds in its cytoplasm. Transformation of appropriate host cells with a vector comprising a nucleic acid sequence according to the invention is accomplished by well known methods that typically depend on the type of vector used. With regard to these methods, see for example, Maniatis et al. 1982, Molecular Cloning, A laboratory Manual, Cold Spring Harbor Laboratory and commercially available methods.
A wide variety of prokaryotic host cells with no mutation allowing formation of disulfide bonds in their cytoplasm is useful in expressing the nucleic acid sequences of this invention. These hosts may include strains of gram-negative cells such as E. coli and Pseudomonas, or gram positive cells such as Bacillus and Streptomyces. Preferably, the host cell is a gram-negative cell, more preferably an E. coli cell. E. coli which can be used are e. g. the strains BL21, TG1, W3110, DH1, XL1-Blue and Origami, or derivates thereof which are commercially available or can be obtained via the DSMZ (Deutsche Sammlung von Mikroorganismen und Zellkulturen GmbH, Braunschweig, Germany). Most preferably, BL 21 (Novagen) or derivates thereof are used.

In another aspect, the invention is directed to a method of detecting and/or quantifying autoantibodies produced in response to an autoimmune disease associated with an immune reaction to a TSH-receptor in a biological fluid of a subject comprising
a) contacting said biological fluid with one or more unglycosylated isolated and purified recombinant polypeptides and/or one or more unglycosylated TSH receptor partial sequences and/or one or more epitopes of the unglycosylated TSH receptor partial sequences of the present invention, and
b) detecting and/or quantifying said autoantibodies bound to said unglycosylated isolated and purified recombinant polypeptides and/or to said unglycosylated TSH receptor partial sequences and/or to said epitopes.

A preferred method of the present invention comprises a) contacting said biological fluid with one or more unglycosylated isolated and purified recombinant polypeptides and one or more epitopes of the unglycosylated TSH receptor partial sequences of the present invention, and
b) detecting and/or quantifying said autoantibodies to said unglycosylated isolated and purified recombinant polypeptides and to said epitopes.

The method according to the invention may be performed as an assay. Procedures and reagents for detecting and/or quantifying autoantibodies produced in response to an autoimmune disease associated with an immune reaction to a TSH-receptor according to the method of the invention which employ the unglycosylated isolated and purified recombinant polypeptides and/or the unglycosylated TSH receptor partial sequences and/or the epitopes of the unglycosylated TSH receptor partial sequences of the present invention in an assay may be conventional, and will admit of design and selection by the skilled person who understands the present disclosure, mindful of well accepted principles of diagnostic and immunodiagnostic methodology and interpretation. Such principles are set forth, for example, in Harlow, E., and Lane, D., "Antibodies: A Laboratory Manual," Cold Spring Harbor Laboratory, Cold Spring Harbor, NY, 349 (1988), Rose, Noel R., et al., eds., Manual of Clinical Laboratory Immunology, Fifth Edition, ASM Press, Washington D. C. (1997), and prior editions ; Rich, R. et al., eds. , Clinical Immunology, Mosby, Publisher, St. Louis (1995).
The types of assays which can be used by practising the method of the present invention and which can be incorporated in kit form are many, and include, for example, indirect assays or direct assays, with indirect assays presently preferred as well as competitive and non-competitive assays, with non-competitive assays presently preferred. Typical examples of assays which can utilize the unglycosylated isolated and purified recombinant polypeptides, the unglycosylated TSH receptor partial sequences and/or the epitopes of the unglycosylated TSH receptor partial sequences and/or one or more epitopes of the unglycosylated TSH receptor partial sequences of the invention are enzyme-linked immunosorbent assays (ELISA) also referred to as enzyme immunoassays (EIA), Luminescense immunosorbent assays (LISA), radioimmunoassays (RIA), Western blot assays, Immunoprecipitation assays as well as flow cytometric assays. These kinds of assays comprise as well as immunometric or sandwich immunoassays. By the term "immunometric assay" or "sandwich immunoassay," it is meant to include simultaneous sandwich, forward sandwich and reverse sandwich immunoassays. These terms are well understood by those skilled in the art. Those of skill will also appreciate that, the isolated and purified recombinant polypeptides, the TSH receptor partial sequences and the epitopes of the unglycosylated TSH receptor partial sequences according to the invention will be useful in other variations and forms of assays which are presently known or which may be developed in the future. These are intended to be included within the scope of the present invention. Usually, the assay of the present invention is selected from ELISA, LISA, RIA, Western Blot, Immunoprecipitation or Flow Cytometrie. Preferably an ELISA, more preferably a non-competitive ELISA, most preferably an indirect non-competitive ELISA is used.
A non-limiting example of a preferred indirect assay according to the invention is described herein, for example, in Example 8, which describes how human serum samples from patients and International Standards may be assayed indirectly for autoantibodies against human TSHR using the unglycosylated isolated and purified recombinant polypeptides of the present invention and a secondarily detecting labelled antibody.

Usually, a label is used for detecting and/or quantifying the autoantibodies against TSHR. There are many different labels and methods of labeling known in the art, however, which can be used in the present invention. Examples of the types of labels include, but are not limited to, enzymes, radioisotopes, fluorescent compounds, chemiluminescent compounds and bioluminescent compounds. Those of ordinary skill in the art will know of other suitable labels, or will be able to ascertain the same by the use of routine experimentation. Furthermore, the binding of these labels to antigens and antibody complex can be accomplished using standard techniques commonly known to those of ordinary skill in the art.

The autoantibodies produced in response to an autoimmune disease associated with an immune reaction to a TSH-receptor are usually detected and/or quantified by the method according to the present invention by labelling the unglycosylated isolated and purified recombinant polypeptides and/or the unglycosylated TSH receptor partial sequences of the present invention and/or antibodies like autoantibodies which bind to the TSH receptor partial sequences and/or the isolated and purified recombinant polypeptides and/or the epitopes of the unglycosylated TSH receptor partial sequences and/or to secondary antibodies which bind to these autoantibodies. Preferably, a secondary antibody which binds to autoantibodies produced in response to an autoimmune disease associated with an immune reaction to a TSH-receptor is labelled. This is usually accomplished by linking the compound to be labelled to an enzyme. This enzyme, in turn, when later exposed to its substrate, will react with the substrate in such a manner as to produce a chemical moiety which can be detected as, for example, by colorimetric, spectrophotometric or fluorometric means. Examples of enzymes which can be used to detectably label antibodies include malate dehydrogenase, staphylococcal nuclease, delta-V-steroid isomerase, yeast alcohol dehydrogenase, alpha-glycerophosphate dehydrogenase, triose phosphate isomerase, biotin-avidin peroxidase, horseradish peroxidase, alkaline phosphatase, asparaginase, glucose oxidase, betagalactosidase, ribonuclease, urease, catalase, glucose-VI-phosphate dehydrogenase, glucoamylase and acetylcholine esterase.

As well a radioactive isotope which then can be determined by such means as the use of a gamma counter or scintillation counter can be used as label. Isotopes in which are particularly useful for the purpose of the present invention are ¹²⁵I, ³H, ³²P, ³⁵P, ³⁵S, ¹⁴C, ⁵¹Cr, ³⁶Cl, ⁵⁷Co, ⁵⁸Co, ⁵⁹Fe and ⁷⁵Se. For in vivo, in vitro or in situ diagnosis, labels such as radiolabels may be used directly or by using and intermediary functional group. An intermediary group which is often used to bind radioisotopes which exist as metallic cations to anti-bodies is diethylenetriaminepentaacetic acid (DTPA). Typical examples of metallic cations which are bound in this manner are: ^{99m}Tc, ¹²³I, ¹¹¹IN, ¹³¹I, ⁹⁷Ru, ⁶⁷Cu, ⁶⁷Ga and ⁶⁸Ga. For labelling as well non-radioactive isotopes for purposes of diagnosis can be used. Elements which are particularly useful in this manner are ¹⁵⁷Gd, ⁵⁵Mn, ¹⁶²Dy, ⁵³Cr and ⁵⁶Fe. It is also possible to use a fluorescent compound as label. When a fluorescently labeled antibody is exposed to light of the proper wave length, its presence then can be detected due to the fluorescence of the dye. Among the most commonly used fluorescent labeling compounds are fluoroscein, isothiocyanate, rhodamine, phycoerythrin, phycocyanin, allophycocyanin, o-phthaldehyde and fluorescamine. As well fluorescent emitting metals such as ¹⁵²Eu, or others of the anthanide series can be used for labelling. These metals can be attached to e. g. the antibody molecule using such metal chelating groups as diethylenetriaminepentaacetic acid (DTPA) or ethylenediaminetetraacetic acid (EDTA). Another useful label is a chemiluminescent compound. The presence of the chemiluminescent-tagged antigen or antibody is then determined by detecting the presence of luminescence that arises during the course of the chemical reaction. Examples of particularly useful chemiluminescent labeling compounds are luminal, isoluminol, aromatic acridinium ester, imidazole, acridinium salts, oxalate ester, and dioxetane. Likewise, a bioluminescent compound may be used for labelling. Bioluminescence is a type of chemiluminescence found in biological systems in which a catalytic protein increases the efficiency of the chemiluminescent reaction. The presence of a bioluminescent antigen or antibody is determined by detecting the presence of luminescence. Important bioluminescent compounds for purposes of labeling include luciferase and aequorin.

Autoantibodies produced in response to an autoimmune disease associated with an immune reaction to a TSH-receptor can be detected/quantified in a biological fluid of a subject such as serum, plasma, lymph, urine and saliva in vivo, in situ or in vitro. Preferably serum is used. Preferably it is detected/quantified in vitro, wherein the biological fluid is obtained as a sample from the subject. Conventional methods for obtaining a sample from a subject known to a person skilled in the art can be used. Preferably the biological fluid sample is contacted with one or more unglycosylated isolated and purified recombinant polypeptides and/or one or more unglycosylated TSH receptor partial sequences and/or one or more epitopes of the unglycosylated TSH receptor partial sequences immobilised on a solid support. Suitable solid supports and procedures of immobilisation which can be used are described below. In case the isolated and purified recombinant polypeptides and/or the TSH receptor partial sequences and/or the epitopes of the unglycosylated TSH receptor partial sequences are immobilised on a solid support autoantibodies bound to said solid support are detected and/or quantified.
Thus, a preferred method of the present invention is a method of detecting and/or quantifying autoantibodies produced in response to an autoimmune disease associated with an immune reaction to a TSH-receptor in a biological fluid of a subject, wherein the biological fluid is obtained as a sample from said subject comprising
a) contacting said sample with one or more unglycosylated isolated and purified recombinant polypeptides and/or one or more unglycosylated TSH receptor partial sequences and/or one or more epitopes of the unglycosylated TSH receptor partial sequences f the present invention immobilised on a solid support, and
b) detecting and/or quantifying said autoantibodies bound to said solid support.

The method of detecting and/or quantifying autoantibodies of the present invention might further comprise the detection and/or quantification of autoantibodies against thyroglobulin (TG) and/or thyroperoxidase (TPO). This can be accomplished by using commercially available epitopes of TG and/or TPO which can be e. g. immobilised on a solid support with the unglycosylated isolated and purified recombinant polypeptides and/or the unglycosylated TSH receptor partial sequences and/or the epitopes of the present invention.

The present method preferably further comprises
c) isolating the detected and/or quantified autoantibodies produced in response to an autoimmune disease associated with an immune reaction to a TSH-receptor. Methods of isolating autoantibodies detected in an assay are known to the person skilled in the art and are described e. g. by Warren K.G. and Catz I., J Neurol Sci. 1991; 103 (1): 90-96.

The autoantibodies produced in response to an autoimmune disease associated with an immune reaction to a TSH-receptor detected and/or quantified by the method of the invention are of the isotypes IgD, IgE, IgG, IgM and/or IgA. Preferably, they are of the isotypes IgG, IgM and/or IgA. The present invention is the first description of the detection of an IgM type anti-TSHR antibody.

Thus, a further object of the present invention are autoantibodies produced in response to an autoimmune disease associated with an immune reaction to a TSH-receptor of the isotypes IgD, IgE, IgG, IgM and/or IgA obtainable by or obtained by the above described method. Preferred are autoantibodies produced in response to an autoimmune disease associated with an immune reaction to a TSH-receptor of the isotypes IgG, IgM and/or IgA, whereas IgM is most preferred.

The unglycosylated isolated and purified recombinant polypeptides and/or the unglycosylated TSH receptor partial sequences and/or the epitopes of the unglycosylated TSH receptor partial sequences of the present invention are ideally suited for the preparation of a kit. Therefore another object of the present invention is to provide a kit for detecting and/or quantifying autoantibodies produced in response to an autoimmune disease associated with an immune reaction to a TSH-receptor in a biological fluid of a subject comprising at least one or more unglycosylated isolated and purified recombinant polypeptides and/or one or more unglycosylated TSH receptor partial sequences and/or one or more epitopes of the unglycosylated TSH receptor partial sequences of the present invention, optionally with reagents and/or instructions for use. Such a kit may comprise further the labels and the labelled compounds as described above in relation to the method of the invention. Such a kit may comprise as well a carrier means being compartmentalized to receive in close confinement therewith one or more container means such as plates, vials, tubes and the like, each of said container means comprising the separate elements of the assay to be used.

The kit can be applied to a biological fluid of a subject such as serum, plasma, lymph, urine and saliva in vivo, in situ or in vitro. Preferably serum is used. Preferably it is applied in vitro, wherein the biological fluid is obtained as a sample from the subject.

The types of assays which can be used by practising the method of the present invention and which can be incorporated in kit form are many. Typical examples of assays which can utilize the unglycosylated isolated and purified recombinant polypeptides and the unglycosylated TSH receptor partial sequences and/or the epitopes of the unglycosylated TSH receptor partial sequences of the invention are described supra.
Thus, the kit of the present invention preferably further comprises reagents for carrying out an assay such as an indirect assay or direct assays preferably an indirect assay, as well as a competitive and non-competitive assay, preferably a non-competitive assay. The assay comprises reagents for carrying out an assay usually selected from ELISA, LISA, RIA, Western blot, Immunoprecipitation as well as Flow Cytometrie. Preferably, the kit includes reagents for carrying out an ELISA, more preferably a non-competitive ELISA, most preferably an indirect non-competitive ELISA. Such reagents are well known to the person skilled in the art and comprise e. g. Immobilization-buffer, Blocking-buffer, Incubation-buffer, Wash-buffer, Substrate-buffer and/or Secondary-Antibody-Conjugate.

There are many solid supports which can be used as immunoadsorbents for the method and the kit of the present invention. Well known materials which may be employed include glass, polystyrene, polypropylenes dextran, nylon, agarose, dextran, acrylamide, Nitrocellulose, PVDF and other materials, in the form of tubes, beads, membranes and microtiter plates formed from or coated with such materials, and the like. The isolated and purified recombinant polypeptides and/or the TSH receptor partial sequences and/or the epitopes of the unglycosylated TSH receptor partial sequences of the present invention can be either covalently or physically bound to the solid support, by techniques such as covalent bonding via an amide, ester or disulfide linkage, or by adsorption. Those skilled in the art will know many other suitable solid support immunoadsorbents and methods for immobilizing antibodies thereon. This binding can be accomplished by using e. g. covalent bonding via an amide, ester or disulfide linkage between the solid support and the isolated an and/or the epitopes of the unglycosylated TSH receptor partial sequences of the present invention. In case the polypeptide to be fused is GST, the isolated and purified recombinant polypeptides is preferably immobilised in such a way that it is aligned on the solid support via a disulfide linkage between the solid support presenting glutathione on its surface and the GST portion of the polypeptide. Presently preferred for use as a solid support are micro titer plates made of polystyrole which can be obtained from various commercial suppliers such as NUNC, Costar, Greiner, Falcon or Coring Inc.
In case the method of detecting and/or quantifying autoantibodies against TSHR is performed on a solid support, usually, the solid support is coated with the unglycosylated isolated and purified recombinant polypeptides and/or the unglycosylated TSH receptor partial sequences and/or the epitopes of the unglycosylated TSH receptor partial sequences of the present invention and/or with antibodies like autoantibodies which bind to the unglycosylated isolated and purified recombinant polypeptides and/or the unglycosylated TSH receptor partial sequences and/or the epitopes of the unglycosylated TSH receptor partial sequences by using a coating buffer known to the person skilled in the art such as PBS buffer or carbonate buffer. Such coating buffers as well as solid supports already coated might be included as reagents to the kit of the present invention.

In a preferred mode for performing the above described method of the invention it is important to use certain "blockers" which might be included as a reagent in the kit of the invention as well. The "blockers" are added to assure that non-specific proteins, protease, or human antibodies other than autoantibodies produced in response to a TSH receptor present in the experimental sample do not cross-link or destroy the antigens or antibodies on the solid support, or the radiolabeled indicator antigen or antibody, to yield false positive or false negative results. A usual blocker which can be used is Bovine Serum Albumin (BSA), which is preferred. The blocker can be added in buffer solution like PBS buffer. In case a solid support is used the blocker is usually added after coating the solid support.

Prior to contacting a biological fluid with the unglycosylated isolated and purified recombinant polypeptides and/or the unglycosylated TSH receptor partial sequences and/or the epitopes of the unglycosylated TSH receptor partial sequences, the biological fluid and/or the unglycosylated isolated and purified recombinant polypeptides and/or the unglycosylated TSH receptor partial sequences and/or the epitopes of the unglycosylated TSH receptor partial sequences can be preteated with the polypeptide fused to the TSH receptor partial sequences in order to avoid false positive or false negative results.

The kit of the present invention might further comprise reagents for carrying out a point of care test. Point of care tests and reagents comprised are known to the person skilled in the art and are e.g. test strips, test sticks or bio-CDs.

The kit of the present invention might further comprise reagents for detecting and/or quantifying TG and TPO. Usual reagents for detecting and/or quantifying TG and TPO are commercially available epitopes of TG and/or TPO which can be e. g. immobilised on a solid support with the isolated and purified recombinant polypeptides and/or the TSH receptor partial sequences and/or the epitopes of the unglycosylated TSH receptor partial sequences of the present invention.

Besides solid-liquid phase assay formats as well liquid-liquid phase assay formats may be employed for the method and the kit of the present invention. Liquid-liquid phase assay formats offer improved reaction kinetics as compared to solid-liquid phase assay formats. In a particular embodiment, the TSH receptor partial sequences and/or the isolated and purified recombinant polypeptides and/or the epitopes of the unglycosylated TSH receptor partial sequences of the present invention may be used to detect autoantibodies produced in response to an autoimmune disease associated with an immune reaction to a TSH-receptor, using an automated diagnostic system such as a microsystem platform suitable to manipulate the sample.

The kit of the present invention is preferably used for the diagnosis of an autoimmune disease associated with an immune reaction to a TSH receptor. An autoimmune disease associated with an immune reaction to a TSH receptor refers to a disease in which the respective subject raises antibodies against the TSH receptor. Typically, the autoimmune disease associated with an immune reaction to a TSH receptor is Graves' disease.

The present invention is also directed to a pharmaceutical composition comprising as an active substance a pharmaceutically effective amount of one or more unglycosylated isolated and purified recombinant polypeptides and/or one or more unglycosylated TSH receptor partial sequences and/or one or more epitopes of the unglycosylated TSH receptor partial sequences of the present invention, optionally in combination with a pharmaceutically acceptable carrier, diluent or adjuvant.

"A pharmaceutically effective amount" refers to a chemical material or compound which, when administered to a human or animal organism induces a detectable pharmacologic and/or physiologic effect. The respective pharmaceutically effect amount can depend on the specific patient to be treated, on the disease to be treated and on the method of administration. Further, the pharmaceutically effective amount depends on the specific compound used.
In addition to one or more unglycosylated TSH receptor partial sequences and/or one or more unglycosylated isolated and purified recombinant polypeptides and/or one or more epitopes of the unglycosylated TSH receptor partial sequences as described herein, the pharmaceutical composition may contain one or more pharmaceutically acceptable carriers, diluents and adjuvants.
Acceptable carriers, diluents and adjuvants which facilitates processing of the active compounds into preparation which can be used pharmaceutically are non-toxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN®, PLURONICS® or polyethylene glycol (PEG).
The form of administration of the pharmaceutical composition may be systemic or topical. For example, administration of such a composition may be various parenteral routes such as subcutaneous, intravenous, intradermal, intramuscular, intraperitoneal, intranasal, transdermal, buccal routes or via an implanted device, and may also be delivered by peristaltic means. The pharmaceutical composition can be in any suitable form, e.g. in the form of a solution, suspension, powder, lyophilisate, ointment or tincture. Preservatives, stabilisers, buffers, antioxidants and/or other additives may be included, as required.

As well encompassed by the present invention is one or more unglycosylated isolated and purified recombinant polypeptides and/or one or more unglycosylated TSH receptor partial sequences and/or one or more epitopes of the unglycosylated TSH receptor partial sequences and/or the pharmaceutical composition of the present invention, for use in the therapeutic treatment or prevention of and/or for use in the diagnosis of autoimmune disease associated with an immune reaction to a TSH receptor.

Furthermore, the use of one or more unglycosylated isolated and purified recombinant polypeptides and/or one or more unglycosylated TSH receptor partial sequences and/or one or more epitopes of the unglycosylated TSH receptor partial sequences and/or the pharmaceutical composition of the present invention, for the preparation of a medicament for the therapeutic treatment or prevention of autoimmune disease associated with an immune reaction to a TSH receptor is encompassed by the present invention.
The therapeutic treatment of autoimmune disease associated with an immune reaction to a TSH receptor are usually carried out by administering to the subject a therapeutically effective amount of one or more unglycosylated isolated and purified recombinant polypeptides and/or one or more unglycosylated TSH receptor partial sequences and/or the pharmaceutical composition of the present invention.

A further object of the present invention is a method of treating autoimmune disease associated with an immune reaction to a TSH receptor in a subject comprising administering to the subject a therapeutically effective amount of a therapeutic agent identified as providing a therapeutic effect by binding with one or more unglycosylated isolated and purified recombinant polypeptides and/or one or more unglycosylated TSH receptor partial sequences and/or one or more epitopes of the unglycosylated TSH receptor partial sequences and/or the pharmaceutical composition of the present invention.
A "therapeutically effective amount" is an amount effective to ameliorate or prevent the symptoms, or prolong the survival of the subject being treated. Determination of a therapeutically effective amount is well within the capabilities of those skilled in the art. For systemic administration, a therapeutically effective amount or dose can be estimated initially from in vitro assays. For example, a dose can be formulated in animal models to achieve a circulating concentration range that includes the IC50 as determined in cell culture. Such information can be used to more accurately determine useful doses in humans. Initial doses can also be estimated from in vivo data, e.g. animal models, using techniques that are well known in the art. One ordinarily skill in the art could readily optimise administration to humans based on animal data and will, of course, depend on the subject being treated, on the subject's weight, the severity of the disorder, the manner of administration and the judgement of the prescribing physician.

Yet another object of the present invention is a method for identifying a binding partner for a TSH receptor, comprising the steps of
a) contacting said binding partner with one or more unglycosylated isolated and purified recombinant polypeptides and/or one or more unglycosylated TSH receptor partial sequences and/or one or more epitopes of the unglycosylated TSH receptor partial sequences, and
b) detecting and/or quantifying the binding partner bound to said unglycosylated isolated and purified recombinant polypeptides and/or said unglycosylated TSH receptor partial sequences and/or said epitopes of the unglycosylated TSH receptor partial sequences
c) optionally isolating the detected and/or quantified binding partner bound to said unglycosylated isolated and purified recombinant polypeptides and/or to said unglycosylated TSH receptor partial sequences and/or said epitopes of the unglycosylated TSH receptor partial sequences.

Procedures and reagents for this method may be conventional, and will admit of design and selection by skilled person who understands the present disclosure, mindful of well accepted principles of analytical and diagnostic methodology and interpretation. Such principles are set forth, for example, in Harlow, E., and Lane, D., "Antibodies: A Laboratory Manual," Cold Spring Harbor Laboratory, Cold Spring Harbor, NY, 349 (1988). The binding partner to be identified can be an antibody like an antibody directed to the TSH receptor, the antigen binding domain of an antibody (Fab), F(ab)2, a single chain antibody as well as a recombinant antibody. Usually, it is an antibody directed to the TSH receptor. The isolated antibody can be further characterized by conventional methods like binding to its antigen TSHR by ELISA, Western Blot, Flow cytometry or by sequencing of it's CDR region.

Thus, a further object of the present invention is a binding partner for a TSH receptor obtainable by this method which binding partner does not comprise TSH. Preferably the binding partner binds to the TSH receptor with a binding constant (Kd) higher than the binding constant of the TSH receptor with monoclonal anti-TSHR antibody 2C11 and/or to monoclonal anti-TSHR antibody 4C1. Binding constants (Kd) can be determined by e. g. fitting specific binding by free ligand concentration by non-linear regression and biacore analysis. The analysis can be done by commercially available data software. Preferably, the binding partner obtainable by this method is an antibody of the IgD, IgE, IgG, IgM or IgA isotype, more preferably an antibody of the IgG, IgM or IgA isotype.

### Examples

### Example 1

### DNA and peptide sequence of the human TSH receptor used to produce TSHR-ED, TSHR-210, TSHR-310

The sequences TSHR-ED, TSHR-210, TSHR-310 refer to the human TSH receptor as available under GeneBank accession number M73747 (GI:903759). The amino acid sequence of the human Thyrotropin Receptor is as follows:

The corresponding cDNA sequence reads as follows:

1 taatacgact cactataggc gaattaagcg atttcggagg atggagaaat agccccgagt
61 cccgtggaaa atgaggccgg cggacttgct gcagctggtg ctgctgctcg acctgcccag
121 ggacctgggc ggaatggggt gttcgtctcc accctgcgag tgccatcagg aggaggactt
181 cagagtcacc tgcaaggata ttcaacgcat ccccagctta ccgcccagta cgcagactct

241 gaagcttatt gagactcacc tgagaactat tccaagtcat gcattttcta atctgcccaa
301 tatttccaga atctacgtat ctatagatgt gactctgcag cagctggaat cacactcctt
361 ctacaatttg agtaaagtga ctcacataga aattcggaat accaggaact taacttacat
421 agaccctgat gccctcaaag agctccccct cctaaagtcc ttggcatttt caaacactgg
481 acttaaaatg ttccctgacc tgaccaaagt ttattccact gatatattct ttatacttga
541 aattacagac aacccttaca tgacgtcaat ccctgtgaat gcttttcagg gactatgcaa
601 tgaaaccttg acactgaagc tgtacaacaa tggctttact tcagtccaag gatatgattt
661 ctttgggaca aagctggatg ctgtttacct aaacaagaat aaatacctga cagttattga
721 caaagatgca tttggaggag tatacagtgg accaagcttg ctggacgtgt ctcaaaccag
781 tgtcactgcc cttccatcca aaggcctgga gcacctgaag gaactgatag caagaaacag
841 ctggactctt aagaaacttg cactttcctt gagtttcctt cacctcacac gggctgacct
901 ttcttaccca agccactgct gtgcttttaa gaatcagaag aaaatcagag gaatccttga
961 gtccttgatg tgtaatgaga gcagtatcga gacgttgcgc cagagaaaat ctgtgaatgc
1021 cttgaatagc cccctccacc aggaatatga agagaatctg ggtgacagca ttgttgggta
1081 caaggaaaag tccaagttcc aggatactca taacaacgct cattattacg tcttctttga
1141 agaacaagag gatgagatca ttggttttgg ccaggagctc aaaaaccccc aggaagagac
1201 tctacaagct tttgacagcc attatgacta caccatatgt ggggacagtg aagacatggt
1261 gtgtaccccc aagtccgatg agttcaaccc gtgtgaagac ataatgggct acaagttcct
1321 gagaattgtg gtgtggttcg ttagtctgct ggctctcctg ggcaatgtct ttgtcctgct
1381 tattctcctc accagccact acaaactgaa cgtcccccgc tttctcatgt gcaacctggc
1441 ctttgcggat ttctgcatgg ggatgtacct gctcctcatc gcctctgtag acctctacac
1501 tcactctgag tactacaacc atgccatcga ctggcagaca ggccctgggt gcaacacggc
1561 tggtttcttc actgtctttg caagcgagtt atcggtgtat acgctgacgg tcatcaccct
1621 ggagcgctgg tatgccatca ccttcgccat ggccctggac cggaagatcc gcctcaggca
1681 cgcatgtgcc atcatggttg ggggctgggt ttgctgcttc cttctcgccc tgcttccttt
1741 ggtgggaata agtagctatg ccaaagtcag tatctgcctg cccatggaca ccgagacccc
1801 tcttgctctg gcatatattg tttttgttct gacgctcaac atagttgcct tcgtcatcgt
1861 ctgctgctgt tatgtgaaga tctacatcac agtccgaaat ccgcacaacc caggggacaa

1921 agataccaaa attgccaaga ggatggctgt gttgatcttc accgacttca cgtgcatggc
1981 cccaatctca ttctatgctg tgtcagcaat tctgaacaag cctctcatca ctgttagcaa
2041 ctccaaaatc ttgctggtac tcttctatcc aattaactcc tgtgccaatc cattcctcta .
2101 tgctattttc accaaggcct tccagaggga tgtgttcatc ctactcagca agtttggcat
2161 ctgtaaacgc caggctcagg cataccgggg gcagagggtt cctccaaaga acagcactga
2221 tattcaggtt caaaaggtta cccacgacat gaggcagggt ctccacaaca tggaagatgt
2281 ctatgaactg attgaaaact cccatctaac cccaaagaag caaggccaaa tctcagaaga
2341 gtatatgcaa acggttttgt aagttaacac tacactactc acaatggtag gggaacttac
2401 aaaataatag ttt

The preferred extracellular portion of the human TSH-Receptor comprises the region from Ntd. 134-1315; Aa 22-415. The sequence of the signal peptide is not included. The TSHR sequence which was used for the experiments presented here refers to accession number M73747 (GI:903759).

### Example 2

### Constructs and cloning procedure

The sequences of the TSHR-Variants presented (TSHR-ED, TSHR-210, TSHR-310) were directly fused to the C-Terminus of Glutathione S-Transferase (GST) from Schistosoma Japonicum using the commercially available vector pGEX-2T from Amersham Biosciences. The GST sequence refers to the GeneBank accession U13850. In the cloning procedure the TSHR-Variants were, according to the restriction sites in the PCR primers, cloned with their 5'portion into the BamHI-site of the pGEX-2T multicloning site and with their 3'portion into the EcoRI-site of this vector. According to this cloning procedure 6 additional amino acids (Glutamate (E) and Phenylalanine (F), Isoleucine (I), Valine (V), Threonine (T) and Aspartic acid (D) bold, green, are fused to the very C-terminal lysine (K) of the extracellular portion of the TSHR protein. Directly after this additional amino acids the reading frame is terminated via a tga STOP-Codon encoded by the vector.

### TSHR-fusion protein 1: GST-TSHR-ED (Vector: pGEX2T-TSHR-ED)

The extracellular domain of the human TSH-receptor was amplified with means of the primers MM38, (5'BamHI: TCTCGGATCCATGGGGTGTTCGTCTCCA) and MM39 (3'-EcoRI: ATATGAATTCCTTGTAGCCCATTATGTCTTCAC) from a human thyroid cDNA pool(Invitrogen) and upon BamHI/EcoRI restriction cloned in frame in the vector pGEX-2T (GE Healthcare, Amersham Biosciences) restricted with the same enzymes resulting in a GST-TSHR-ED fusion construct (GST = Glutathion-S-Transferase) corresponding to SEQ ID No. 4 (Aa) and corresponding to SEQ ID No. 7 (Ntd.). The cloned TSHR sequence spans the region from Ntd. 134-1315 (Aa 22-415 corresponding to SEQ ID No. 1).

### TSHR-fusion protein 2: GST-TSHR-210(Vector: pGEX2T-TSHR-210)

This variant of the human TSH-receptor was amplified with means of the primers MM126, 5'BamHI: CGCGGATCCAATAAATACCTGACAGTTATTGACAAA) and MM39 (3'-EcoRI: ATATGAATTCCTTGTAGCCCATTATGTCTTCAC) using pGEX2T-TSHR-ED as a template. The amplification product was, upon BamHI/EcoRI restriction, cloned in frame in the vector pGEX-2T (GE Healthcare, Amersham Biosciences) restricted with the same enzymes resulting in a GST-TSHR-210 fusion construct (GST = Glutathion-S-Transferase) corresponding to SEQ ID No. 5 (Aa) and corresponding to SEQ ID No. 8 (Ntd.). The cloned TSHR sequence spans the region from Ntd. 698-1315 (Aa 210-415 corresponding to SEQ ID No. 2).

### Variant 3: GST-TSHR-310 (Vector: pGEX2T-TSHR-310)

This variant of the human TSH-receptor was amplified with means of the primers MM127, 5'BamHI:CGCGGATCCCGCCAGAGAAAATCTGTGAA) and MM39 (3'-EcoRl: and was, upon BamHI/EcoRI restriction, cloned in frame in the vector pGEX-2T (GE Healthcare, Amersham Biosciences) restricted with the same enzymes resulting in a GST-TSHR-310 fusion construct (GST = Glutathion-S-Transferase) corresponding to SEQ ID No. 6 (Aa) and corresponding to SEQ ID No. 9 (Ntd.). The cloned TSHR sequence spans the region from Ntd. 998-1315 (Aa 210-415 corresponding to SEQ ID No. 3).

### Example 3

### Purification of the recombinant GST-TSHR-fusion proteins

Expression and purification of the various TSHR-variants was performed according to the following experimental procedure.
a. Fresh Transformation of pGEX2T-TSHR vectors (ED, 210, 310) in the bacterial strain BL21 (typically 10ng plasmid DNA/200 µl competent bacteria)
b. O/n culture under selection pressure (ampicilline 100 µg/ml final concentration)
c. Dilution of the o/n culture (1/20) and cultivation until logarithmical growth of the culture
d. Induction of the target gene by addition of IPTG (final concentration: 1 mM).
e. Induction culture for further 4h
f. Isolation of the bacteria by centrifugation
g. Resuspension and wash of the bacteria in Tris buffered saline (TBS: 50mM Tris pH 8.0, 150 mM NaCl)
h. Resuspension of the bacteria in Lysis/Solubilsation buffer (TBS, 1% Triton X100, 1mM DTT, 1mM EDTA)
i. Disruption of the bacteria by sonification (lysate preparation)
j. Solubilization of fusion proteins by incubation/agitation of the bacterial lysate (30min/4°C)
k. Separation of insoluble components of the bacterial lysate by high speed centrifugation
l. Purification of GST-TSHR fusion proteins from the bacterial lysate (supernatant from step k) by affinity chromatography using Glutathione-Sepharose (GE Healthcare, formerly Amersham Pharmacia Biotech) as affinity matrix.
m. Elution of GST-TSHR fusion proteins using 20mM reduced Glutathione in TBS
n. Dialysis of the eluate (against TBS)
o. Determination of size and purity of purified fusion proteins by SDS-PAGE/Coomassie stain
p. Quality control by Western Blot Analysis using a polyclonal anti-GST antiserum and Monoclonal anti-TSHR antibodies 2C11 or 4C1 (Serotec)
q. Photometric determination of protein concentration (OD 280)

The isolated and purified TSHR-Variants in TBS are (upon adequate dilution) "the ready to use samples" for the further experimental procedures.

### Analysis of the GST-TSHR-Variants by SDS-PAGE and Coomassie stain

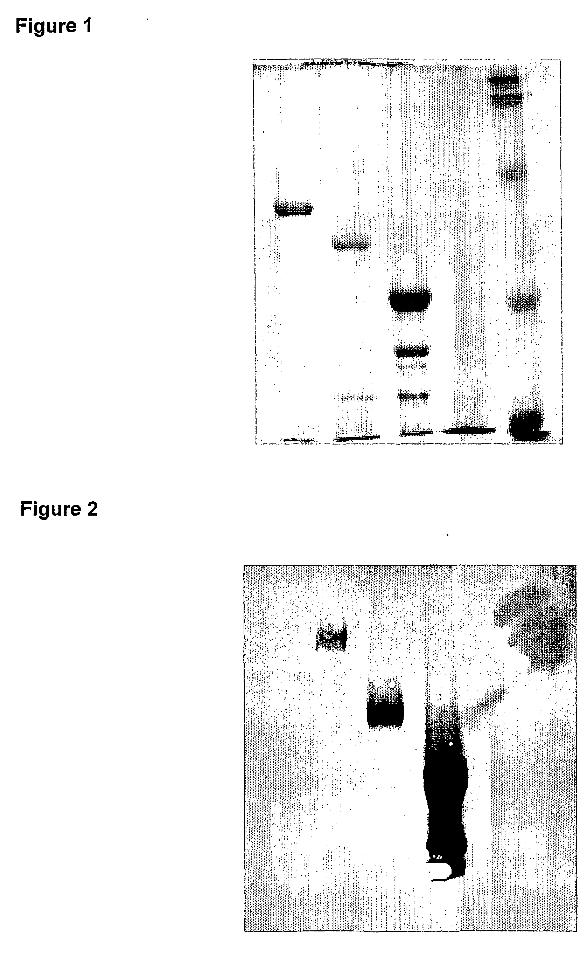

Upon chromatographic purification and dialysis proteins were boiled/reduced in protein sample buffer and subsequenlty subjected to SDS-PAGE (10% SDS-PAG). The staining was performed with Coomassie Brilliant Blue according to a standard protocol as shown in Figure 1. From left to the right: TSHR-ED, TSHR-210, TSHR-310, blank, Protein Standard.

### Analysis of the GST-TSHR-Variants by Western Blotting

After SDS-PAGE proteins were transferred from the gel onto a nitrocellulose membrane. Specific proteins were detected with the TSHR-specific monoclonal antibody 2C11 and an adequate secondary antibody/substrate system (Rabbit anti Mouse-HRP, DINOVA, and Metal enhanced DAB, PIERCE) as shown in Figure 2 (From left to the right: TSHR-ED, TSHR-210, TSHR-310).

### Example 4

### Coating TSH receptor variants on micro-well plate

Affinity purified TSH receptor variants were diluted with PBS buffer in suitable concentration. 120 µl of diluted TSH receptor in PBS coating buffer were then added to plastic wells of microtiter plate and incubated at room temperature for at least 6 hours or overnight. After discarding the coating buffer, 150 µl of blocking buffer (1 % BSA in PBS buffer) will be added to each microwell and incubated at room temperature for another 30 min to overcome the unspecific binding reaction. The blocking buffer will then be discarded and the microtiter plates were dried at 37°C for 2 hour for immediately using for ELISA or sealed in aluminum foil bag with dry pad for storage at 4°C.

### Example 5

### Analysis of the activities of E.coli expressed TSH receptor variants

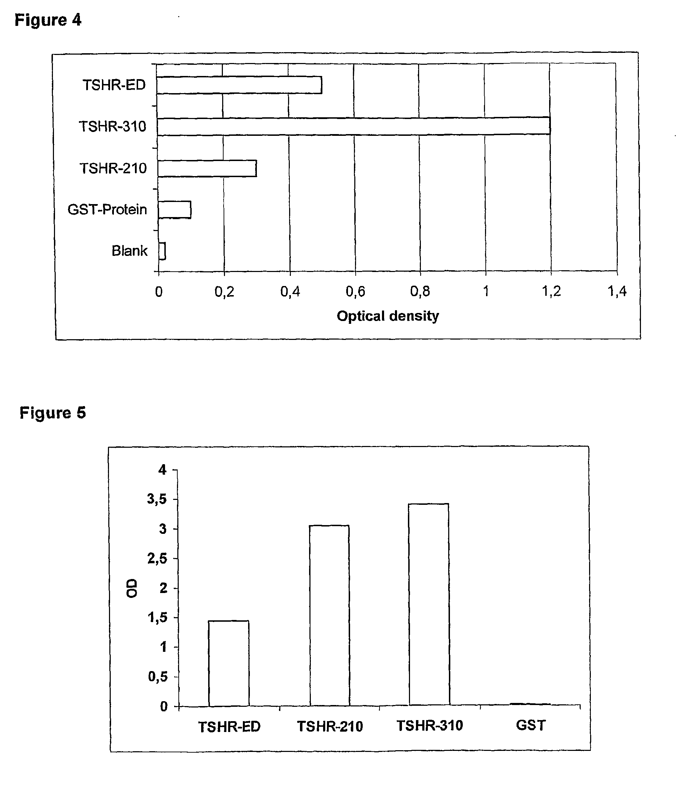

a. Binding activity to TSH
   To determine the physiological activity of the purified GST-TSHR proteins it was investigated whether they can bind to their physiological ligand TSH. TSH from bovine thyroid (Sigma, St. Louis) was immobilized on a ELISA Plate (NUNC, Wiesbaden). Free binding sites were blocked by incubation with a 3% solution of bovine serum albumine. Subsequently the indicated GST-TSHR-Varients and a appropriate control (the GST protein alone) were incubated on the plate and the binding was visualized with Rabbit anti-GST (Signa, St. Louis) in combination with Goat anti Rabbit-Peroxidase (DIANOVA) and TMB as a peroxidase substrate. The results are shown in Figure 4.
b. Binding activity to mouse produced TSH receptor antibodies
   Mouse produced TSH receptor antibodies 4C1 were used which recognise the human thyroid stimulating hormone (TSH) receptor by binding to the extracellular domain, according to the manufacture instruction. The affinity of TSHR-variants (from example 3) immobilized in plastic wells of microtiter plate (from example 4) were used to test the binding activity to mouse anti-TSHR antibodies. The mouse anti-TSHR antibodies were diluted in suitable concentration according to the manufacture instruction in PBS buffer. 100 µl of antibodies mixture were added to the plastic wells and incubated at room temperature for 30 minutes. After discarding the antibodies mixture, the wells were washed three times with suitable amounts of PBS buffer. The binding activity of affinity purified TSHR fusion protein to mouse anti-TSHR antibodies were detected by horseradish peroxidases-conjugated Rabbit anti-Mouse IgG (Figure 5). In order to avoid false positive, GST with same concentration as TSHR variants was immobilized in plastic well to serve as a negative control.

### Example 6

### Detection of TRAbs (TSH receptor autoantibodies) in positive TSHR sera

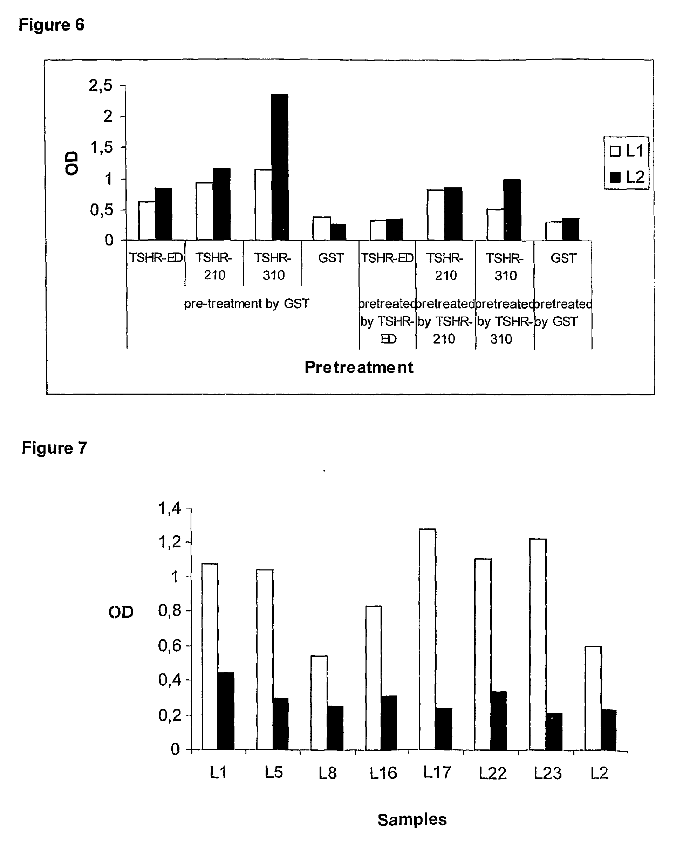

Commercial available TSHR positive sera L1 and L2 (Invent Diagnostic GmbH) were diluted in PBS buffer. L1 and L2 are positive anti-TSHR sera, pooled from different patient and with low and high activities according to the manufacturer. The TSHR-variants, TSHR-ED, -210 and -310 are immobilized in the plastic wells of microtiter plate according to example 4. The samples were pre-treated with GST or with affinity purified TSHR fusion protein for 30 min, then 100µl of diluted sample were added to the well for incubation at room temperature for 30 minutes. The affinity of autoantibodies bound to TSHR-variants was secondarily detected by measuring the signal from rabbit anti-human immunoglobulins linked horseradish peroxidase oxidizing the substrate (TMB, tetramethylbenzidine) measured at 450 nm against 620 nm as reference (Figure 6). The specificity of this binding activity can be demonstrated by pre-treating the human sera with TSHR-variants causing the loss of signal.

### Example 7

### Detection of TRAbs (TSH receptor autoantibodies) in human sera by using THSR-variants immobilized microtiter plate

TRAbs from patient sera and pooled commercial sera (L2, Invent Diagnostic GmbH) were detected with THSR-variants immobilized on microtiter plate (example 4) with suitable coating concentration. Patient sera as well as L2 commercial available sera were diluted with PBS buffer and 100µl of sample, and then incubated with TSHR-variants immobilized on microtiter plate. The detection of anti-TSHR autoantibodies in patient sera were demonstrated by the specific signal response as processed at example 6. The results are shown in Figure 7, in which blue bars indicate that sera above are processed with purified GST protein and rot bars with purified TSHR variants.

### Example 8

### Detection of TRAbs in human sera by comparing the signal with international standard 90/672

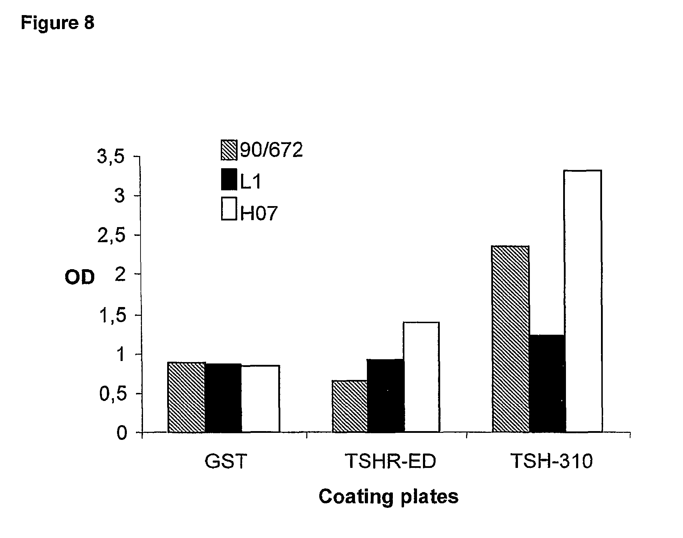

90/672 is an international standard defined as 100 mili-international units of TSH stimulating antibodies per ampoule. This standard as well as L1 commercial available sera and H07 (patient serum) were processed as example 6, but without pretreating, to compare their signal responses as shown in Figure 8. GST and TSHR-ED coated microtiter wells gave minimum signal in all of three sera, but strong signals in TSHR-310 coated microtiter wells were obtained.

### Example 9

### Detection of different isoforms of TRAbs in human sera

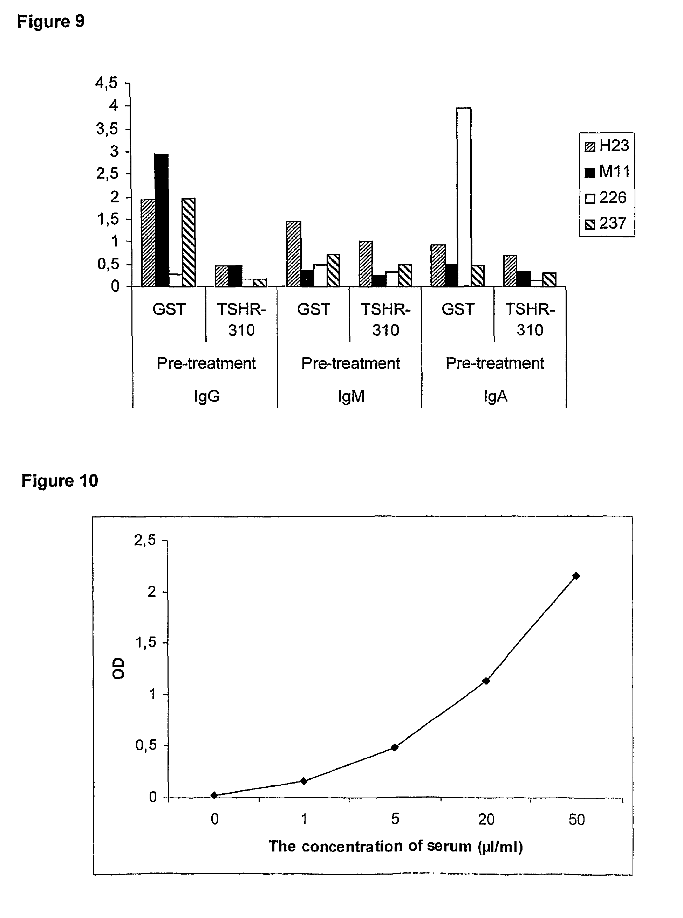

The isoforms of TRAbs in human sera were detected with the procedure as described in example 7, the specific response of IgG, IgM and IgA were defined with specific rabbit anti-human isoforms of immunoglobins directed to IgG, IgM or IgA linked with horseradish peroxidase (Figure 9).

### Example 10

### Dose-dependent Reaction of TSHR-variants to anti-TSHR autoantibodies

The commercial available serum (L2, Invent Diagnostic GmbH) was diluted, and the signal response to a mixture of TSHR-ED and TSHR-310 (around 50:50) coated microtiter plate was measured by ELISA as described at example 6. The results of the different dilutions are shown in Figure 10.

### Example 11

### Cleavage of the GST-TSHR-fusion protein TSHR-ED with Thrombin

Purified GST-TSHR-ED was incubated with various amounts of Thrombin (1 h, 37°C), subsequently boiled in SDS-sample buffer and subjected to SDS-PAGE/Western Blotting. TSHR-ED and fragments therof were visualized with anti-TSHR mAb 2C11 and appropiate secondary reagents. Lanes:
1. Prestained Marker: 200, 119, 66, 43, 20, 14,3 kD
2. GST-TSHR-ED, without Thrombin
3. GST-TSHR-ED, with 0.1 U Thrombin
4. GST-TSHR-ED, with 0.5U Thrombin
5. GST-TSHR-ED, with 1 U Thrombin
6. GST-TSHR-ED, with 2U Thrombin

### Example 12

### Coating of GST-tagged TSHR recombinant protein through glutathione immobilized microwells

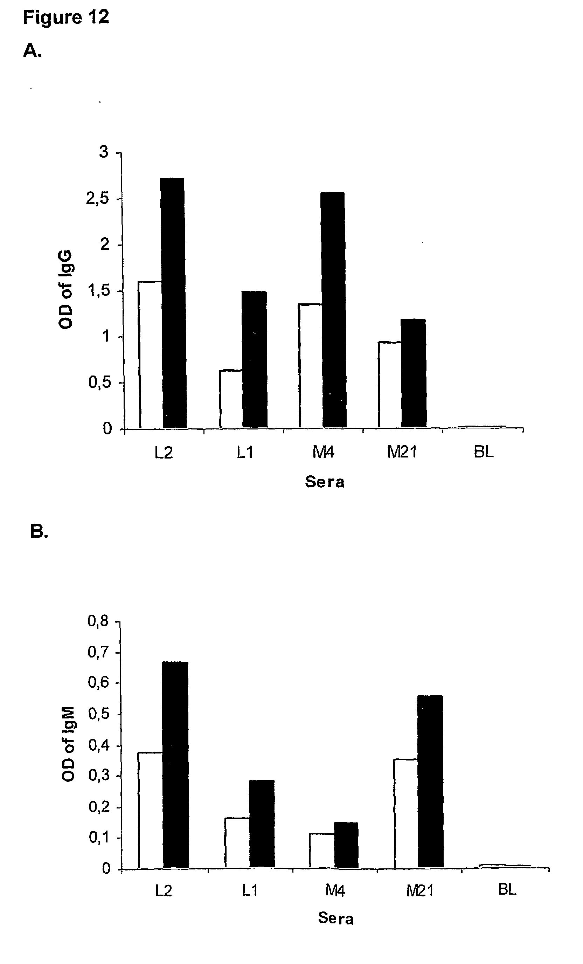

Affinity purified GST-TSHR-ED protein is diluted with PBS buffer to a suitable concentration and coated directly on microtiter plae (Corning) as described previously or through glutathione immobilized microtiter plates (NUNC) according to manufacture instruction. The binding of anti-TSHR autoantibodies IgG or IgM to GST-TSHR-ED was detected as described in example 6. As Fig. 12 shows, the binding activities of GST-TSHR-ED to both IgG and IgM are higher in GST-TSHR-ED coated through glutathione immobilized plates than GST-TSHR-ED ditectly coated plates. The experiments show that at the same coating concentration, the binding activity of GST-TSHR-ED protein to its autoantibodies IgG(A) and IgM(B) in 4 tsted patient sera (L2, L1, M4 and M21) is significantly higher in GST-TSHR-ED coating through glutathione immobilized microtiterplace than GST-TSHR-ED directly coated plate.

### Example 13

### Binding activity of GST-TSHR-ED to anti-TSHR autoantibodies is reduced in denatured GST-TSHR-ED recombinant protein coated microtiter plate

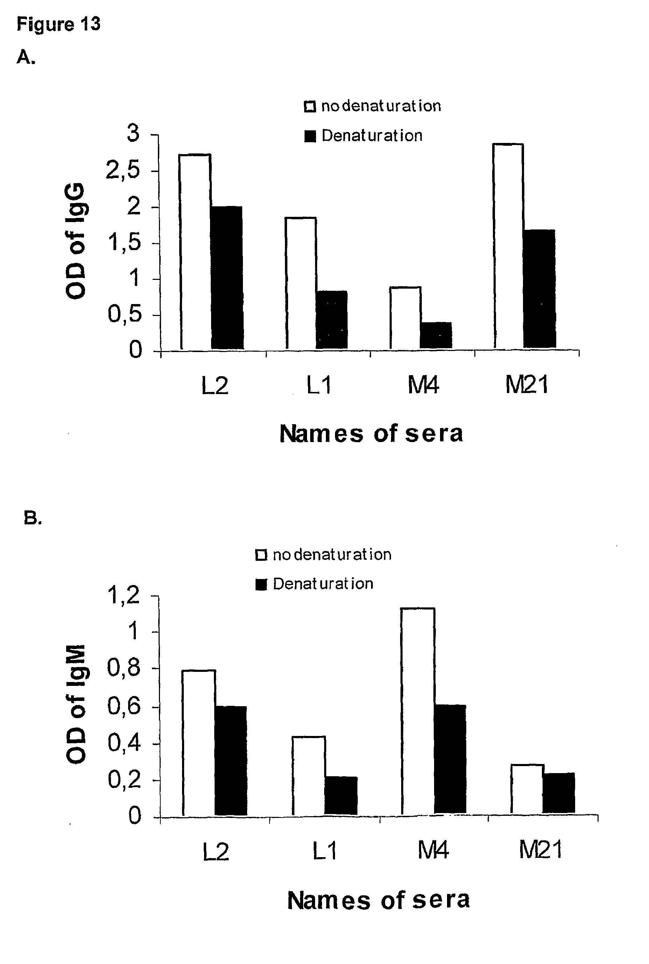

Affinity purified GST-TSHR-ED were coated on microtiter plates in the same concentration without protein denaturation and with cooking of 5 minutes in water bath to denature the GST-TSHR-ED protein. Anti-TSHR autoantibodies IgG and IgM in 4 patient sera were tested in the above coated plates. A reduced binding activity of GST-TSHR-ED to autoantibodies IgG(A) and IgM(B) is observed after GST-TSHR-ED is denatured by cooking in water bath for 5 minutes before coating (Fig. 13).

### Example 14

### Identification of TSHR-specific autoantibody epitopes by TSHR-specific Peptide Scanning

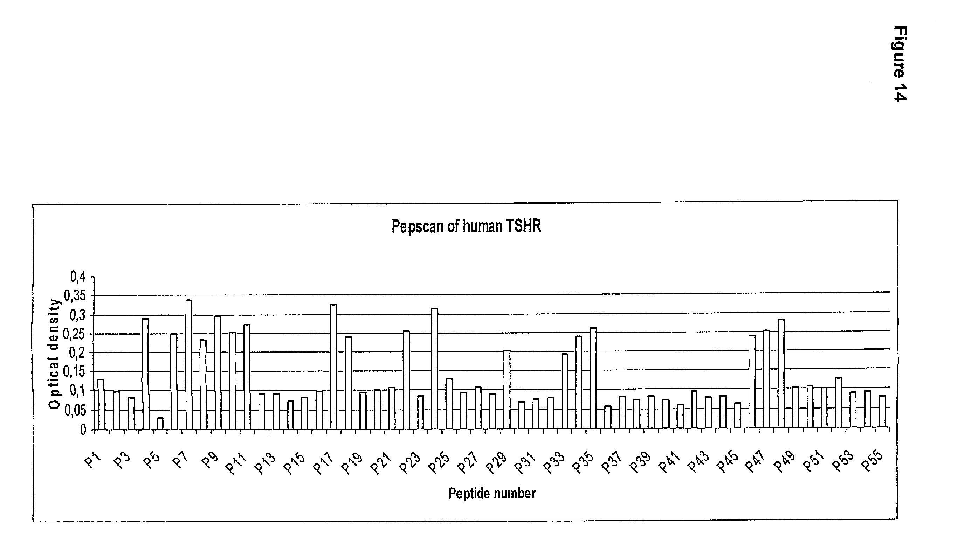

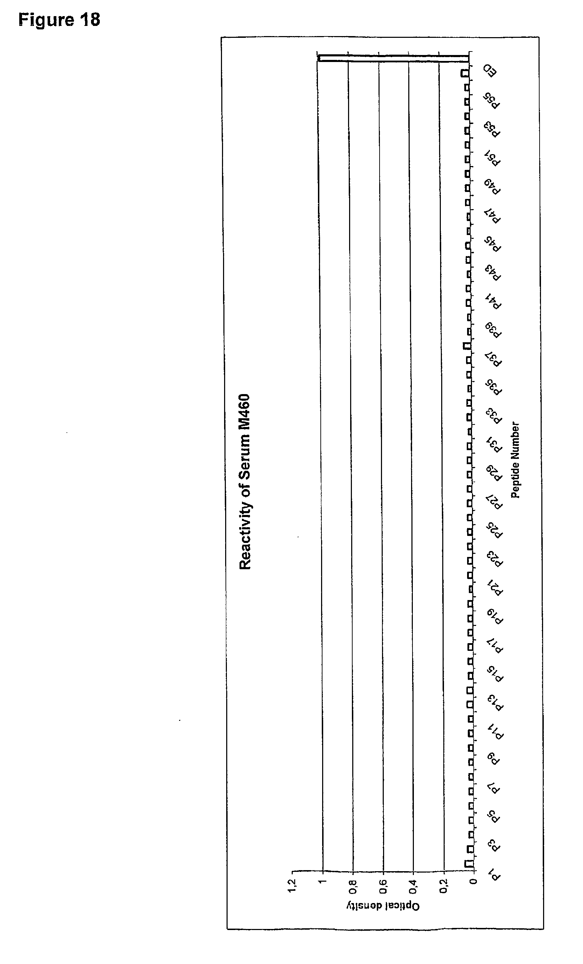

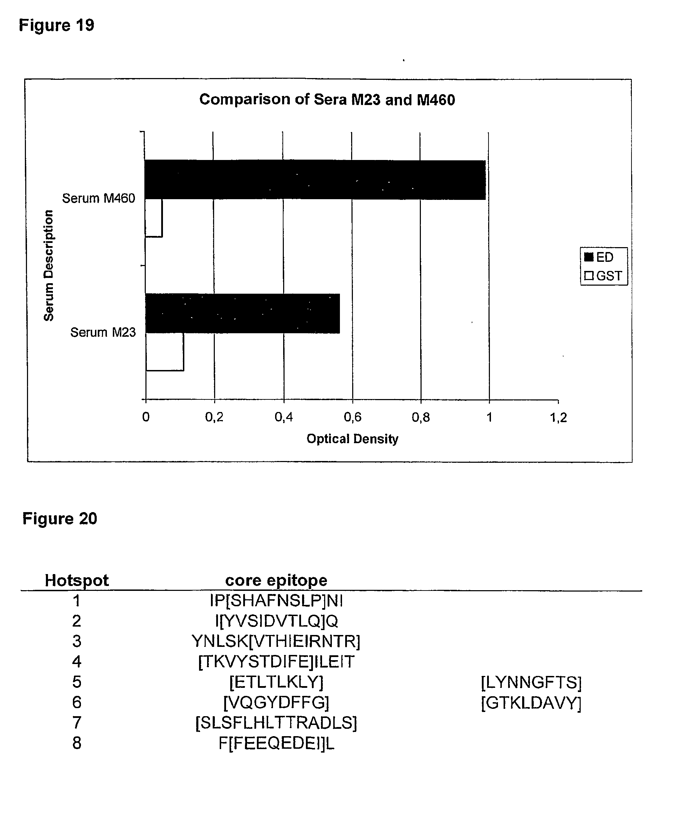

To identify epitopes reacting with TSHR specific autoantibodies a TSHR specific peptide scanning method was implemented. In brief 55 20mer peptides were synthesized with a overlap of 7 amino acids (aa) covering the entire extracellular domain of the human TSHR from aa22 to aa415.The peptides were immobilized onto ELISA plates and subjected to ELISA-assay with individual human serum samples. Using this method a series (n=8) of autoimmunogenic linear epitopes of the human TSHR could be identified (Figures 15, 16 and 17). Figure 14 shows the reactivity of a individual serum (Serum M23) reacting with the peptides. Therefore it can be predicted that there are TSHR specific autoantibodies from patient sera which react with epitopes of the TSHR. However it was also found out that there is a series of sera reacting strongly with the recombinant GST-TSHR-ED produces in bacteria but not with the individual peptides (Example: Serum460, Figs. 18/19). Although Serum460 reacts with recombinant GST-TSHR-ED in a comparable manner to Serum M23 it does not bind to TSHR specific peptides (Figs. 18/19). Therefore it can be predicted that the GST-TSHR-ED expressed in the bacterial sytem of the present invention displays TSHR specific conformational epitopes and that individual patients samples contain autoantibodies recognizing linear as well as conformational epitopes of the human TSHR. The ratio as well as the overall amount of these autoantibodies determines whether a serum sample reacts with the epitopes displayed by the TSHR-specific peptide set and the bacterially expressed GST-TSHR-ED or with the recombinant GST-TSHR-ED alone.

### Example 16

### PepScan of a individual serum sample recognizing epitopes (Serum M23)

The TSHR-peptides were immobilized onto a NUNC Maxisorb ELISA plate (5µg/ml, o/n) and non specific binding sites were saturated (3 % BSA). The serum sample was diluted 1/100 in TBS/1%BSA and subsequently pipetted in all wells. Autoantibodies reacting with the peptides were visualized with HRP conjugated Rabbit anti Mouse-Ig (DIANOVA, Hamburg) at a dilution of 1:50.000 an the appropriate substance (POD-Blue, Roche), as shown in Fig. 14. The analysis of 38 patient's sera is shown in Fig. 15 and the determination of reactivity of serum M460 as shown in Fig. 18 were performed accordingly.

### Example 17

### Comparison of Serum 23 with Serum 460

Recombinant proteins (GST-TSHR-ED as well as the negative vontrol GST) were immobilized onto a NUNC Maxisorb ELISA plate (5µg/ml, o/n) and non specific binding sites were saturated (3 % BSA). The serum samples were diluted 1/100 in TBS/1%BSA and subsequently pipetted onto the coated wells. Autoantibodies reacting with the recombinant proteins were visualized with HRP conjugated Rabbit anti Mouse-Ig (DIANOVA, Hamburg) at a dilution of 1:50.000 an the appropriate substrate (POD-Blue, Roche), as shown in Fig. 19.

### Example 18

### Core epitopes of the identified hotspots

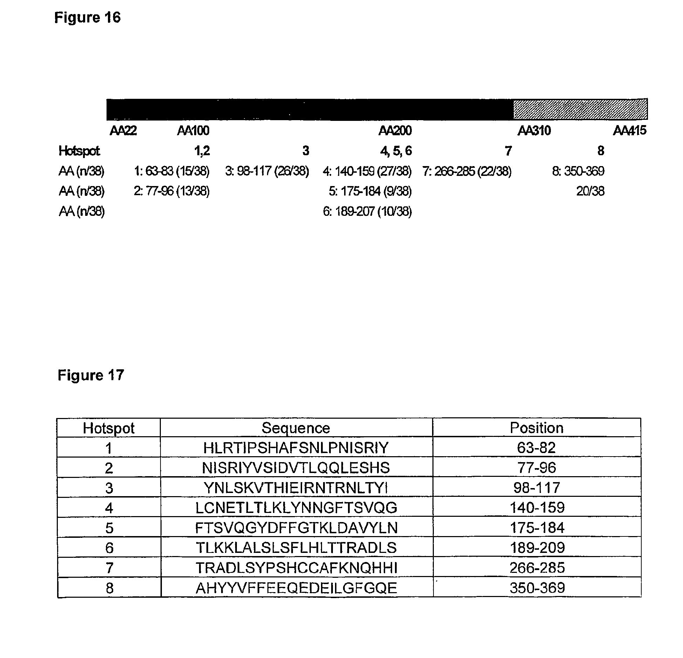

The core epitopes of the autoreactive hotspots as shown in Fig. 17 could be narrowed down by means of the TSHR spedific epitope scan method (Fig. 20). Sequences in square brackets represent the preferred minimal epitope sequences, amino acids surrounding the sequences in square brackets might be important for optimal binding of TSHR specific autoantibodies. For hotspots 5/6 two peptides could be relevant.

## Claims

1. An epitope of an unglycosylated TSH receptor partial sequence selected from the group consisting of
a) an epitope comprising at least 5 consecutive amino acids of the amino acid sequence selected from SEQ ID Nos: 10-27;
b) an epitope comprising an amino acid sequence selected from SEQ ID Nos: 10-27;
c) an epitope which is at least 60 % identical to the amino acid sequences as defined in a) or b).

2. A method of detecting and/or quantifying autoantibodies produced in response to an autoimmune disease associated with an immune reaction to a TSH-receptor in a biological fluid of a subject comprising
a) contacting said biological fluid with
(i) one or more unglycosylated isolated and purified recombinant polypeptides comprising a fusion protein able to bind to autoantibodies produced in response to an autoimmune disease associated with an immune reaction to a TSH receptor obtainable by the method comprising a) transforming a prokaryotic host with no mutation allowing formation of disulfide bonds in the cytoplasm of the prokaryotic host cells with a vector comprising an isolated and purified nucleic acid sequence encoding said fusion protein, wherein one or more TSH receptor partial sequences is fused to a polypeptide and wherein said TSH receptor partial sequences consist essentially of the extracellular domain of the TSH receptor, fragments thereof or variants thereof,
b) culturing said prokaryotic host cells whereby said fusion protein is expressed and accumulated in said prokaryotic host cells
c) isolating said prokaryotic host cells
d) lysing said prokaryotic host cells in the presence of a detergent
e) separating soluble components of the lysate from insoluble components
f) purifying said fusion protein from said soluble components of said lysate;
(ii) an unglycosylated recombinant polypeptide expressed in a prokaryotic host with no mutation allowing formation of disulfide bonds in the cytoplasm of the prokaryotic host cells, said unglycosylated isolated and purified recombinant polypeptide comprising a fusion protein able to bind to autoantibodies produced in response to an autoimmune disease associated with an immune reaction to a TSH-receptor, wherein one or more TSH receptor partial sequences is fused to a polypeptide consisting essentially of the glutathione S transferase (GST), fragments thereof or variants thereof, and wherein said TSH receptor partial sequence consists essentially of the extracellular domain of the TSH receptor, fragments thereof or variants thereof;
(iii) one or more unglycosylated TSH receptor partial sequences able to bind to autoantibodies produced in response to an autoimmune disease associated with an immune reaction to a TSH-receptor, said unglycosylated TSH receptor partial sequences consisting essentially of the extracellular domain of the TSH receptor, fragments thereof or variants thereof obtainable by the method comprising,
a) transforming a prokaryotic host with no mutation allowing formation of disulfide bonds in the cytoplasm of the prokaryotic host cells with a vector comprising an isolated and purified nucleic acid sequence encoding a fusion protein able to bind to auto-antibodies produced in response to an autoimmune disease associated with an immune reaction to a TSH-receptor, wherein one or more TSH receptor partial sequences is fused to a polypeptide and wherein said TSH receptor partial sequences consist essentially of the extracellular domain of the TSH receptor, fragments thereof or variants thereof,
b) culturing said prokaryotic host cells whereby said fusion protein is expressed and accumulated in said prokaryotic host cells
c) isolating said prokaryotic host cells
d) lysing said prokaryotic host cells in the presence of a detergent
e) separating soluble components of the lysate from insoluble components
f) purifying said fusion protein from said soluble components of said lysate
g) digesting or cleaving said polypeptide fused to said TSH receptor partial sequence
h) recovering said TSH receptor partial sequence, fragments thereof or variants thereof;
and/or one (iv) or more epitopes of claim 1;
and
b) detecting and/or quantifying said autoantibodies bound to said unglycosylated isolated and purified recombinant polypeptides and/or to said unglycosylated TSH receptor partial sequences and/or to said epitopes.

3. The method of claim 2, wherein the autoantibodies produced in response to an autoimmune disease associated with an immune reaction to a TSH-receptor are detected/quantified in vitro, wherein the biological fluid is obtained as a sample from the subject.

4. The method of claim 2 or 3, wherein the biological fluid is obtained as a sample from the subject, and wherein said sample is contacted with the unglycosylated isolated and purified recombinant polypeptides and/or the unglycosylated TSH receptor partial sequences and/or the epitopes immobilised on a solid support, and wherein autoantibodies bound to said solid support are detected and/or quantified.

5. The method of claim 4, wherein the sample is contacted with the unglycosylated isolated and purified recombinant polypeptides, wherein one or more THS receptor partial sequences is fused to a polypeptide consisting essentially of glutathione S transferase (GSHT), fragments thereof or variants thereof ans wherein said fusion protein is immobilized in such a way that it is aligned on the solid support via a disulfide linkage with the solid support presenting glutathione on its surface.

6. The method of claim 2 to 5, wherein the autoantibodies are detected and/or quantified according to an assay selected from ELISA, LISA, RIA, Western Blot, Immunoprecipitation or Flow Cytometrie.

7. The method of claim 6, wherein the assay is an ELISA.

8. The method of claims 2-7, wherein the detected and/or quantified autoantibodies are of the isotypes IgG, IgM and/or IgA.

9. The method of claims 2-8, further comprising
c) isolating the detected and/or quantified autoantibodies.

10. The method of claims 2-9, further detecting and/or quantifying autoantibodies against TG and/or TPO.

11. A kit for detecting and/or quantifying autoantibodies produced in response to an autoimmune disease associated with an immune reaction to a TSH-receptor in a biological fluid of a subject comprising at least
(i) one or more unglycosylated isolated and purified recombinant polypeptides comprising a fusion protein able to bind to autoantibodies produced in response to an autoimmune disease associated with an immune reaction to a TSH receptor obtainable by the method comprising a) transforming a prokaryotic host with no mutation allowing formation of disulfide bonds in the cytoplasm of the prokaryotic host cells with a vector comprising an isolated and purified nucleic acid sequence encoding said fusion protein, wherein one or more TSH receptor partial sequences is fused to a polypeptide and wherein said TSH receptor partial sequences consist essentially of the extracellular domain of the TSH receptor, fragments thereof or variants thereof,
b) culturing said prokaryotic host cells whereby said fusion protein is expressed and accumulated in said prokaryotic host cells
c) isolating said prokaryotic host cells
d) lysing said prokaryotic host cells in the presence of a detergent
e) separating soluble components of the lysate from insoluble components
f) purifying said fusion protein from said soluble components of said lysate;
(ii) an unglycosylated recombinant polypeptide expressed in a prokaryotic host with no mutation allowing formation of disulfide bonds in the cytoplasm of the prokaryotic host cells, said unglycosylated isolated and purified recombinant polypeptide comprising a fusion protein able to bind to autoantibodies produced in response to an autoimmune disease associated with an immune reaction to a TSH-receptor, wherein one or more TSH receptor partial sequences is fused to a polypeptide consisting essentially of the glutathione S transferase (GST), fragments thereof or variants thereof, and wherein said TSH receptor partial sequence consists essentially of the extracellular domain of the TSH receptor, fragments thereof or variants thereof;
(iii) one or more unglycosylated TSH receptor partial sequences able to bind to auto-antibodies produced in response to an autoimmune disease associated with an immune reaction to a TSH-receptor, said unglycosylated TSH receptor partial sequences consisting essentially of the extracellular domain of the TSH receptor, fragments thereof or variants thereof obtainable by the method comprising,
a) transforming a prokaryotic host with no mutation allowing formation of disulfide bonds in the cytoplasm of the prokaryotic host cells with a vector comprising an isolated and purified nucleic acid sequence encoding a fusion protein able to bind to autoantibodies produced in response to an autoimmune disease associated with an immune reaction to a TSH-receptor, wherein one or more TSH receptor partial sequences is fused to a polypeptide and wherein said TSH receptor partial sequences consist essentially of the extracellular domain of the TSH receptor, fragments thereof or variants thereof,
b) culturing said prokaryotic host cells whereby said fusion protein is expressed and accumulated in said prokaryotic host cells
c) isolating said prokaryotic host cells
d) lysing said prokaryotic host cells in the presence of a detergent
e) separating soluble components of the lysate from insoluble components
f) purifying said fusion protein from said soluble components of said lysate
g) digesting or cleaving said polypeptide fused to said TSH receptor partial sequence
h) recovering said TSH receptor partial sequence, fragments thereof or variants thereof; and/or (iv) one or more epitopes of claim 1;
optionally with reagents and/or instructions for use.

12. The kit of claim 11, further comprising reagents for carrying out an assay selected from ELISA, LISA, RIA, Western Blot, Immunoprecipitation or Flow Cytometrie, preferably wherein the assay is an ELISA; or the kit of claim 11, further comprising reagents for carrying out a point of care test.

13. The kit of claims 11 to 12, further comprising reagents for detecting and/or quantifying TG and TPO.

14. Use of the kit of claims 11 to 13 for the diagnosis of an autoimmune disease associated with an immune reaction to a TSH receptor.

15. One or more
(i) unglycosylated isolated and purified recombinant polypeptides comprising a fusion protein able to bind to autoantibodies produced in response to an autoimmune disease associated with an immune reaction to a TSH receptor obtainable by the method comprising a) transforming a prokaryotic host with no mutation allowing formation of disulfide bonds in the cytoplasm of the prokaryotic host cells with a vector comprising an isolated and purified nucleic acid sequence encoding said fusion protein, wherein one or more TSH receptor partial sequences is fused to a polypeptide and wherein said TSH receptor partial sequences consist essentially of the extracellular domain of the TSH receptor, fragments thereof or variants thereof,
b) culturing said prokaryotic host cells whereby said fusion protein is expressed and accumulated in said prokaryotic host cells
c) isolating said prokaryotic host cells
d) lysing said prokaryotic host cells in the presence of a detergent
e) separating soluble components of the lysate from insoluble components
f) purifying said fusion protein from said soluble components of said lysate;
(ii) an unglycosylated recombinant polypeptide expressed in a prokaryotic host with no mutation allowing formation of disulfide bonds in the cytoplasm of the prokaryotic host cells, said unglycosylated isolated and purified recombinant polypeptide comprising a fusion protein able to bind to autoantibodies produced in response to an autoimmune disease associated with an immune reaction to a TSH-receptor, wherein one or more TSH receptor partial sequences is fused to a polypeptide consisting essentially of the glutathione S transferase (GST), fragments thereof or variants thereof, and wherein said TSH receptor partial sequence consists essentially of the extracellular domain of the TSH receptor, fragments thereof or variants thereof;
(iii) one or more unglycosylated TSH receptor partial sequences able to bind to auto-antibodies produced in response to an autoimmune disease associated with an immune reaction to a TSH-receptor, said unglycosylated TSH receptor partial sequences consisting essentially of the extracellular domain of the TSH receptor, fragments thereof or variants thereof obtainable by the method comprising,
a) transforming a prokaryotic host with no mutation allowing formation of disulfide bonds in the cytoplasm of the prokaryotic host cells with a vector comprising an isolated and purified nucleic acid sequence encoding a fusion protein able to bind to auto-antibodies produced in response to an autoimmune disease associated with an immune reaction to a TSH-receptor, wherein one or more TSH receptor partial sequences is fused to a polypeptide and wherein said TSH receptor partial sequences consist essentially of the extracellular domain of the TSH receptor, fragments thereof or variants thereof,
b) culturing said prokaryotic host cells whereby said fusion protein is expressed and accumulated in said prokaryotic host cells
c) isolating said prokaryotic host cells
d) lysing said prokaryotic host cells in the presence of a detergent
e) separating soluble components of the lysate from insoluble components
f) purifying said fusion protein from said soluble components of said lysate
g) digesting or cleaving said polypeptide fused to said TSH receptor partial sequence
h) recovering said TSH receptor partial sequence, fragments thereof or variants thereof;
and/or one (iv) or more epitopes of claim 1,
for use in the therapeutic treatment or prevention of and/or for use in the diagnosis of autoimmune disease associated with an immune reaction to a TSH receptor.


SEQUENCE LISTING

<110>  Dr. Fenning Biomed GmbH / Micromol GmbH

<120>  Recombinant polypeptides and methods for detecting and/or
       quantifying autoantibodies against TSH receptor

<130>  PC 08 345

<150>  WO2007/057778
<151>  2006-11-21

<160>  27

<170>  PatentIn version 3.3

<210>  1
<211>  394
<212>  PRT
<213>  Homo sapiens

<220>
<221>  amino acid sequence 22 to 415 from the N-terminus of TSH receptor
<222>  (1)..(394)

<400>  1

Met Gly Cys Ser Ser Pro Pro Cys Glu Cys His Gln Glu Glu Asp Phe
1               5                   10                  15

Arg Val Thr Cys Lys Asp Ile Gln Arg Ile Pro Ser Leu Pro Pro Ser
            20                  25                  30

Thr Gln Thr Leu Lys Leu Ile Glu Thr His Leu Arg Thr Ile Pro Ser
        35                  40                  45

His Ala Phe Ser Asn Leu Pro Asn Ile Ser Arg Ile Tyr Val Ser Ile
    50                  55                  60

Asp Val Thr Leu Gln Gln Leu Glu Ser His Ser Phe Tyr Asn Leu Ser
65                  70                  75                  80

Lys Val Thr His Ile Glu Ile Arg Asn Thr Arg Asn Leu Thr Tyr Ile
                85                  90                  95

Asp Pro Asp Ala Leu Lys Glu Leu Pro Leu Leu Lys Ser Leu Ala Phe
            100                 105                 110

Ser Asn Thr Gly Leu Lys Met Phe Pro Asp Leu Thr Lys Val Tyr Ser
        115                 120                 125

Thr Asp Ile Phe Phe Ile Leu Glu Ile Thr Asp Asn Pro Tyr Met Thr
    130                 135                 140

Ser Ile Pro Val Asn Ala Phe Gln Gly Leu Cys Asn Glu Thr Leu Thr
145                 150                 155                 160

Leu Lys Leu Tyr Asn Asn Gly Phe Thr Ser Val Gln Gly Tyr Asp Phe
                165                 170                 175

Phe Gly Thr Lys Leu Asp Ala Val Tyr Leu Asn Lys Asn Lys Tyr Leu
            180                 185                 190

Thr Val Ile Asp Lys Asp Ala Phe Gly Gly Val Tyr Ser Gly Pro Ser
        195                 200                 205

Leu Leu Asp Val Ser Gln Thr Ser Val Thr Ala Leu Pro Ser Lys Gly
    210                 215                 220

Leu Glu His Leu Lys Glu Leu Ile Ala Arg Asn Ser Trp Thr Leu Lys
225                 230                 235                 240

Lys Leu Ala Leu Ser Leu Ser Phe Leu His Leu Thr Arg Ala Asp Leu
                245                 250                 255

Ser Tyr Pro Ser His Cys Cys Ala Phe Lys Asn Gln Lys Lys Ile Arg
            260                 265                 270

Gly Ile Leu Glu Ser Leu Met Cys Asn Glu Ser Ser Ile Glu Thr Leu
        275                 280                 285

Arg Gln Arg Lys Ser Val Asn Ala Leu Asn Ser Pro Leu His Gln Glu
    290                 295                 300

Tyr Glu Glu Asn Leu Gly Asp Ser Ile Val Gly Tyr Lys Glu Lys Ser
305                 310                 315                 320

Lys Phe Gln Asp Thr His Asn Asn Ala His Tyr Tyr Val Phe Phe Glu
                325                 330                 335

Glu Gln Glu Asp Glu Ile Ile Gly Phe Gly Gln Glu Leu Lys Asn Pro
            340                 345                 350

Gln Glu Glu Thr Leu Gln Ala Phe Asp Ser His Tyr Asp Tyr Thr Ile
        355                 360                 365

Cys Gly Asp Ser Glu Asp Met Val Cys Thr Pro Lys Ser Asp Glu Phe
    370                 375                 380

Asn Pro Cys Glu Asp Ile Met Gly Tyr Lys
385                 390

<210>  2
<211>  206
<212>  PRT
<213>  Homo sapiens

<220>
<221>  amino acid sequence 210 to 415 from the N-terminus of TSH
       receptor
<222>  (1)..(206)

<400>  2

Asn Lys Tyr Leu Thr Val Ile Asp Lys Asp Ala Phe Gly Gly Val Tyr
1               5                   10                  15

Ser Gly Pro Ser Leu Leu Asp Val Ser Gln Thr Ser Val Thr Ala Leu
            20                  25                  30

Pro Ser Lys Gly Leu Glu His Leu Lys Glu Leu Ile Ala Arg Asn Ser
        35                  40                  45

Trp Thr Leu Lys Lys Leu Ala Leu Ser Leu Ser Phe Leu His Leu Thr
    50                  55                  60

Arg Ala Asp Leu Ser Tyr Pro Ser His Cys Cys Ala Phe Lys Asn Gln
65                  70                  75                  80

Lys Lys Ile Arg Gly Ile Leu Glu Ser Leu Met Cys Asn Glu Ser Ser
                85                  90                  95

Ile Glu Thr Leu Arg Gln Arg Lys Ser Val Asn Ala Leu Asn Ser Pro
            100                 105                 110

Leu His Gln Glu Tyr Glu Glu Asn Leu Gly Asp Ser Ile Val Gly Tyr
        115                 120                 125

Lys Glu Lys Ser Lys Phe Gln Asp Thr His Asn Asn Ala His Tyr Tyr
    130                 135                 140

Val Phe Phe Glu Glu Gln Glu Asp Glu Ile Ile Gly Phe Gly Gln Glu
145                 150                 155                 160

Leu Lys Asn Pro Gln Glu Glu Thr Leu Gln Ala Phe Asp Ser His Tyr
                165                 170                 175

Asp Tyr Thr Ile Cys Gly Asp Ser Glu Asp Met Val Cys Thr Pro Lys
            180                 185                 190

Ser Asp Glu Phe Asn Pro Cys Glu Asp Ile Met Gly Tyr Lys
        195                 200                 205

<210>  3
<211>  106
<212>  PRT
<213>  Homo sapiens

<220>
<221>  amino acid sequence 310 to 415 from the N-terminus of TSH
       receptor
<222>  (1)..(106)

<400>  3

Arg Gln Arg Lys Ser Val Asn Ala Leu Asn Ser Pro Leu His Gln Glu
1               5                   10                  15


Tyr Glu Glu Asn Leu Gly Asp Ser Ile Val Gly Tyr Lys Glu Lys Ser
            20                  25                  30

Lys Phe Gln Asp Thr His Asn Asn Ala His Tyr Tyr Val Phe Phe Glu
        35                  40                  45

Glu Gln Glu Asp Glu Ile Ile Gly Phe Gly Gln Glu Leu Lys Asn Pro
    50                  55                  60

Gln Glu Glu Thr Leu Gln Ala Phe Asp Ser His Tyr Asp Tyr Thr Ile
65                  70                  75                  80

Cys Gly Asp Ser Glu Asp Met Val Cys Thr Pro Lys Ser Asp Glu Phe
                85                  90                  95

Asn Pro Cys Glu Asp Ile Met Gly Tyr Lys
            100                 105

<210>  4
<211>  626
<212>  PRT

<213>  Artificial

<220>
<223>  recombinant polypeptide

<220>
<221>  GST-TSHR-ED
<222>  (1)..(626)
<223>  (1) ... (626)

<220>
<221>  GST-TSHR-ED
<222>  (1)..(626)
<223>  recombinant polypeptide

<400>  4

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Val Pro Arg
    210                 215                 220

Gly Ser Met Gly Cys Ser Ser Pro Pro Cys Glu Cys His Gln Glu Glu
225                 230                 235                 240

Asp Phe Arg Val Thr Cys Lys Asp Ile Gln Arg Ile Pro Ser Leu Pro
                245                 250                 255

Pro Ser Thr Gln Thr Leu Lys Leu Ile Glu Thr His Leu Arg Thr Ile
            260                 265                 270

Pro Ser His Ala Phe Ser Asn Leu Pro Asn Ile Ser Arg Ile Tyr Val
        275                 280                 285

Ser Ile Asp Val Thr Leu Gln Gln Leu Glu Ser His Ser Phe Tyr Asn
    290                 295                 300

Leu Ser Lys Val Thr His Ile Glu Ile Arg Asn Thr Arg Asn Leu Thr
305                 310                 315                 320

Tyr Ile Asp Pro Asp Ala Leu Lys Glu Leu Pro Leu Leu Lys Ser Leu
                325                 330                 335

Ala Phe Ser Asn Thr Gly Leu Lys Met Phe Pro Asp Leu Thr Lys Val
            340                 345                 350

Tyr Ser Thr Asp Ile Phe Phe Ile Leu Glu Ile Thr Asp Asn Pro Tyr
        355                 360                 365

Met Thr Ser Ile Pro Val Asn Ala Phe Gln Gly Leu Cys Asn Glu Thr
    370                 375                 380

Leu Thr Leu Lys Leu Tyr Asn Asn Gly Phe Thr Ser Val Gln Gly Tyr
385                 390                 395                 400

Asp Phe Phe Gly Thr Lys Leu Asp Ala Val Tyr Leu Asn Lys Asn Lys
                405                 410                 415

Tyr Leu Thr Val Ile Asp Lys Asp Ala Phe Gly Gly Val Tyr Ser Gly
            420                 425                 430

Pro Ser Leu Leu Asp Val Ser Gln Thr Ser Val Thr Ala Leu Pro Ser
        435                 440                 445

Lys Gly Leu Glu His Leu Lys Glu Leu Ile Ala Arg Asn Ser Trp Thr
    450                 455                 460

Leu Lys Lys Leu Ala Leu Ser Leu Ser Phe Leu His Leu Thr Arg Ala
465                 470                 475                 480

Asp Leu Ser Tyr Pro Ser His Cys Cys Ala Phe Lys Asn Gln Lys Lys
                485                 490                 495

Ile Arg Gly Ile Leu Glu Ser Leu Met Cys Asn Glu Ser Ser Ile Glu
            500                 505                 510

Thr Leu Arg Gln Arg Lys Ser Val Asn Ala Leu Asn Ser Pro Leu His
        515                 520                 525

Gln Glu Tyr Glu Glu Asn Leu Gly Asp Ser Ile Val Gly Tyr Lys Glu
    530                 535                 540

Lys Ser Lys Phe Gln Asp Thr His Asn Asn Ala His Tyr Tyr Val Phe
545                 550                 555                 560

Phe Glu Glu Gln Glu Asp Glu Ile Ile Gly Phe Gly Gln Glu Leu Lys
                565                 570                 575

Asn Pro Gln Glu Glu Thr Leu Gln Ala Phe Asp Ser His Tyr Asp Tyr
            580                 585                 590

Thr Ile Cys Gly Asp Ser Glu Asp Met Val Cys Thr Pro Lys Ser Asp
        595                 600                 605

Glu Phe Asn Pro Cys Glu Asp Ile Met Gly Tyr Lys Glu Phe Ile Val
    610                 615                 620

Thr Asp
625

<210>  5
<211>  438
<212>  PRT
<213>  Artificial

<220>
<223>  recombinant polypeptide

<220>
<221>  GST-TSHR-210
<222>  (1)..(438)
<223>  recombinant polypeptide

<400>  5

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Val Pro Arg

210                 215                 220

Gly Ser Asn Lys Tyr Leu Thr Val Ile Asp Lys Asp Ala Phe Gly Gly
225                 230                 235                 240

Val Tyr Ser Gly Pro Ser Leu Leu Asp Val Ser Gln Thr Ser Val Thr
                245                 250                 255

Ala Leu Pro Ser Lys Gly Leu Glu His Leu Lys Glu Leu Ile Ala Arg
            260                 265                 270

Asn Ser Trp Thr Leu Lys Lys Leu Ala Leu Ser Leu Ser Phe Leu His
        275                 280                 285

Leu Thr Arg Ala Asp Leu Ser Tyr Pro Ser His Cys Cys Ala Phe Lys
    290                 295                 300

Asn Gln Lys Lys Ile Arg Gly Ile Leu Glu Ser Leu Met Cys Asn Glu
305                 310                 315                 320

Ser Ser Ile Glu Thr Leu Arg Gln Arg Lys Ser Val Asn Ala Leu Asn
                325                 330                 335

Ser Pro Leu His Gln Glu Tyr Glu Glu Asn Leu Gly Asp Ser Ile Val
            340                 345                 350

Gly Tyr Lys Glu Lys Ser Lys Phe Gln Asp Thr His Asn Asn Ala His
        355                 360                 365

Tyr Tyr Val Phe Phe Glu Glu Gln Glu Asp Glu Ile Ile Gly Phe Gly
    370                 375                 380

Gln Glu Leu Lys Asn Pro Gln Glu Glu Thr Leu Gln Ala Phe Asp Ser
385                 390                 395                 400

His Tyr Asp Tyr Thr Ile Cys Gly Asp Ser Glu Asp Met Val Cys Thr
                405                 410                 415

Pro Lys Ser Asp Glu Phe Asn Pro Cys Glu Asp Ile Met Gly Tyr Lys
            420                 425                 430

Glu Phe Ile Val Thr Asp
        435

<210>  6
<211>  338
<212>  PRT
<213>  Artificial

<220>
<223>  recombinant polypeptide

<220>
<221>  GST-TSHR-310
<222>  (1)..(338)
<223>  recombinant polypeptide

<400>  6

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Val Pro Arg
    210                 215                 220

Gly Ser Arg Gln Arg Lys Ser Val Asn Ala Leu Asn Ser Pro Leu His
225                 230                 235                 240

Gln Glu Tyr Glu Glu Asn Leu Gly Asp Ser Ile Val Gly Tyr Lys Glu
                245                 250                 255

Lys Ser Lys Phe Gln Asp Thr His Asn Asn Ala His Tyr Tyr Val Phe
            260                 265                 270

Phe Glu Glu Gln Glu Asp Glu Ile Ile Gly Phe Gly Gln Glu Leu Lys
        275                 280                 285

Asn Pro Gln Glu Glu Thr Leu Gln Ala Phe Asp Ser His Tyr Asp Tyr
    290                 295                 300

Thr Ile Cys Gly Asp Ser Glu Asp Met Val Cys Thr Pro Lys Ser Asp
305                 310                 315                 320

Glu Phe Asn Pro Cys Glu Asp Ile Met Gly Tyr Lys Glu Phe Ile Val
                325                 330                 335

Thr Asp


<210>  7
<211>  1880
<212>  DNA

<213>  Artificial

<220>
<223>  region encoding recombinant polypeptide

<220>
<221>  sequence encoding GST-TSHR-ED
<222>  (1)..(1880)
<223>  region encoding recombinant polypeptide

<400>  7
atgtccccta tactaggtta ttggaaaatt aagggccttg tgcaacccac tcgacttctt      60

ttggaatatc ttgaagaaaa atatgaagag catttgtatg agcgcgatga aggtgataaa     120

tggcgaaaca aaaagtttga attgggtttg agtttcccaa tcttccttat tatattgatg     180

gtgatgttaa attaacacag tctatggcca tcatacgtta tatagctgac aagcacaaca     240

tgttgggtgg ttgtccaaaa gagcgtgcag agatttcaat gcttgaagga gcggttttgg     300

atattagata cggtgtttcg agaattgcat atagtaaaga ctttgaaact ctcaaagttg     360

attttcttag caagctacct gaaatgctga aaatgttcga agatcgttta tgtcataaaa     420

catatttaaa tggtgatcat gtaacccatc ctgacttcat gttgtatgac gctcttgatg     480

ttgttttata catggaccca atgtgcctgg atgcgttccc aaaattagtt tgttttaaaa     540

aacgtattga agctatccca caaattgata agtacttgaa atccagcaag tatatagcat     600

ggcctttgca gggctggcaa gccacgtttg gtggtggcga ccatcctcca aaatcggatc     660

tggttccgcg tggatccatg gggtgttcgt ctccaccctg cgagtgccat caggaggagg     720

acttcagagt cacctgcaag gatattcaac gcatccccag cttaccgccc agtacgcaga     780

ctctgaagct tattgagact cacctgagaa ctattccaag tcatgcattt tctaatctgc     840

ccaatatttc cagaatctac gtatctatag atgtgactct gcagcagctg gaatcacact     900

ccttctacaa tttgagtaaa gtgactcaca tagaaattcg gaataccagg aacttaactt     960

acatagaccc tgatgccctc aaagagctcc ccctcctaaa gtccttggca ttttcaaaca    1020

ctggacttaa aatgttccct gacctgacca aagtttattc cactgatata ttctttatac    1080

ttgaaattac agacaaccct tacatgacgt caatccctgt gaatgctttt cagggactat    1140

gcaatgaaac cttgacactg aagctgtaca acaatggctt tacttcagtc caaggatatg    1200

atttctttgg gacaaagctg gatgctgttt acctaaacaa gaataaatac ctgacagtta    1260

ttgacaaaga tgcatttgga ggagtataca gtggaccaag cttgctggac gtgtctcaaa    1320

ccagtgtcac tgcccttcca tccaaaggcc tggagcacct gaaggaactg atagcaagaa    1380

acagctggac tcttaagaaa cttgcacttt ccttgagttt ccttcacctc acacgggctg    1440

acctttctta cccaagccac tgctgtgctt ttaagaatca gaagaaaatc agaggaatcc    1500

ttgagtcctt gatgtgtaat gagagcagta tcgagacgtt gcgccagaga aaatctgtga    1560

atgccttgaa tagccccctc caccaggaat atgaagagaa tctgggtgac agcattgttg    1620

ggtacaagga aaagtccaag ttccaggata ctcataacaa cgctcattat tacgtcttct 1680

ttgaagaaca agaggatgag atcattggtt ttggccagga gctcaaaaac ccccaggaag 1740

agactctaca agcttttgac agccattatg actacaccat atgtggggac agtgaagaca 1800

tggtgtgtac ccccaagtcc gatgagttca acccgtgtga agacataatg ggctacaagg 1860

aattcatcgt gactgactga 1880

<210> 8
<211> 1376
<212> DNA
<213> Artificial

<220>
<223> region encoding recombinant polypeptide

<220>
<221> sequence encoding GST-TSHR-210
<222> (1)..(1376)
<223> region encoding recombinant polypeptide

<400> 8
atgtccccta tactaggtta ttggaaaatt aagggccttg tgcaacccac tcgacttctt 60

ttggaatatc ttgaagaaaa atatgaagag catttgtatg agcgcgatga aggtgataaa 120

tggcgaaaca aaaagtttga attgggtttg agtttcccaa tcttccttat tatattgatg 180

gtgatgttaa attaacacag tctatggcca tcatacgtta tatagctgac aagcacaaca 240

tgttgggtgg ttgtccaaaa gagcgtgcag agatttcaat gcttgaagga gcggttttgg 300

atattagata cggtgtttcg agaattgcat atagtaaaga ctttgaaact ctcaaagttg 360

attttcttag caagctacct gaaatgctga aaatgttcga agatcgttta tgtcataaaa 420

catatttaaa tggtgatcat gtaacccatc ctgacttcat gttgtatgac gctcttgatg 480

ttgttttata catggaccca atgtgcctgg atgcgttccc aaaattagtt tgttttaaaa 540

aacgtattga agctatccca caaattgata agtacttgaa atccagcaag tatatagcat 600

ggcctttgca gggctggcaa gccacgtttg gtggtggcga ccatcctcca aaatcggatc 660

tggttccgcg tggatccact tcagtccaag gatatgattt ctttgggaca aagctggatg 720

ctgtttacct aaacaagaat aaatacctga cagttattga caaagatgca tttggaggag 780

tatacagtgg accaagcttg ctggacgtgt ctcaaaccag tgtcactgcc cttccatcca 840

aaggcctgga gcacctgaag gaactgatag caagaaacag ctggactctt aagaaacttg 900

cactttcctt gagtttcctt cacctcacac gggctgacct ttcttaccca agccactgct 960

gtgcttttaa gaatcagaag aaaatcagag gaatccttga gtccttgatg tgtaatgaga 1020

gcagtatcga gacgttgcgc cagagaaaat ctgtgaatgc cttgaatagc cccctccacc 1080

aggaatatga agagaatctg ggtgacagca ttgttgggta caaggaaaag tccaagttcc 1140

aggatactca taacaacgct cattattacg tcttctttga agaacaagag gatgagatca 1200

ttggttttgg ccaggagctc aaaaaccccc aggaagagac tctacaagct tttgacagcc 1260

attatgacta caccatatgt ggggacagtg aagacatggt gtgtaccccc aagtccgatg 1320

agttcaaccc gtgtgaagac ataatgggct acaaggaatt catcgtgact gactga 1376

<210> 9
<211> 1016
<212> DNA
<213> Homo sapiens

<220>
<221> sequence encoding GST-TSHR-312
<222> (1)..(1016)
<223> region encoding recombinant polypeptide

<400> 9
atgtccccta tactaggtta ttggaaaatt aagggccttg tgcaacccac tcgacttctt 60

ttggaatatc ttgaagaaaa atatgaagag catttgtatg agcgcgatga aggtgataaa 120

tggcgaaaca aaaagtttga attgggtttg agtttcccaa tcttccttat tatattgatg 180

gtgatgttaa attaacacag tctatggcca tcatacgtta tatagctgac aagcacaaca 240

tgttgggtgg ttgtccaaaa gagcgtgcag agatttcaat gcttgaagga gcggttttgg 300

atattagata cggtgtttcg agaattgcat atagtaaaga ctttgaaact ctcaaagttg 360

attttcttag caagctacct gaaatgctga aaatgttcga agatcgttta tgtcataaaa 420

catatttaaa tggtgatcat gtaacccatc ctgacttcat gttgtatgac gctcttgatg 480

ttgttttata catggaccca atgtgcctgg atgcgttccc aaaattagtt tgttttaaaa 540

aacgtattga agctatccca caaattgata agtacttgaa atccagcaag tatatagcat 600

ggcctttgca gggctggcaa gccacgtttg gtggtggcga ccatcctcca aaatcggatc 660

tggttccgcg tggatcccgc cagagaaaat ctgtgaatgc cttgaatagc cccctccacc 720

aggaatatga agagaatctg ggtgacagca ttgttgggta caaggaaaag tccaagttcc 780

aggatactca taacaacgct cattattacg tcttctttga agaacaagag gatgagatca 840

ttggttttgg ccaggagctc aaaaaccccc aggaagagac tctacaagct tttgacagcc 900

attatgacta caccatatgt ggggacagtg aagacatggt gtgtaccccc aagtccgatg 960

agttcaaccc gtgtgaagac ataatgggct acaaggaatt catcgtgact gactga 1016

<210> 10
<211> 20
<212> PRT
<213> Homo sapiens

<220>

<221>  TSHR-specific autoantibody epitope
<222>  (1)..(20)

<400>  10

<210>  11
<211>  20
<212>  PRT
<213>  Homo sapiens

<220>
<221>  TSHR-specific autoantibody epitope
<222>  (1)..(20)

<400>  11

Asn Ile Ser Arg Ile Tyr Val Ser Ile Asp Val Thr Leu Gln Gln Leu
1               5                   10                  15

Glu Ser His Ser
            20

<210>  12
<211>  20
<212>  PRT
<213>  Homo sapiens

<220>
<221>  TSHR-specific autoantibody epitope
<222>  (1)..(20)

<400>  12

Tyr Asn Leu Ser Lys Val Thr His Ile Glu Ile Arg Asn Thr Arg Asn
1               5                   10                  15

Leu Thr Tyr Ile
            20

<210>  13
<211>  20
<212>  PRT
<213>  Homo sapiens

<220>
<221>  TSHR-specific autoantibody epitope
<222>  (1)..(20)

<400>  13

Leu Cys Asn Glu Thr Leu Thr Leu Lys Leu Tyr Asn Asn Gly Phe Thr
1               5                   10                  15

Ser Val Gln Gly
            20

<210>  14
<211>  20
<212>  PRT
<213>  Homo sapiens

<220>
<221>  TSHR-specific autoantibody epitope
<222>  (1)..(20)

<400>  14

Phe Thr Ser Val Gln Gly Tyr Asp Phe Phe Gly Thr Lys Leu Asp Ala
1               5                   10                  15

Val Tyr Leu Asn
            20

<210>  15
<211>  21
<212>  PRT
<213>  Homo sapiens

<220>
<221>  TSHR-specific autoantibody epitope
<222>  (1)..(21)

<400>  15

Thr Leu Lys Lys Leu Ala Leu Ser Leu Ser Phe Leu His Leu Thr Thr
1               5                   10                  15

Arg Ala Asp Leu Ser
            20

<210>  16
<211>  20
<212>  PRT
<213>  Homo sapiens

<220>
<221>  TSHR-specific autoantibody epitope
<222>  (1)..(20)

<400>  16

Thr Arg Ala Asp Leu Ser Tyr Pro Ser His Cys Cys Ala Phe Lys Asn
1               5                   10                  15

Gln Lys Lys Ile
            20


<210>  17
<211>  20
<212>  PRT
<213>  Homo sapiens


<220>
<221>  TSHR-specific autoantibody epitope
<222>  (1)..(20)

<400>  17

Ala His Tyr Tyr Val Phe Phe Glu Glu Gln Glu Asp Glu Ile Leu Gly
1               5                   10                  15


Phe Gly Gln Glu
            20


<210>  18
<211>  12
<212>  PRT
<213>  Homo sapiens


<220>
<221>  TSHR-specific autoantibody epitope
<222>  (1)..(12)

<400>  18

Ile Pro Ser His Ala Phe Asn Ser Leu Pro Asn Ile
1               5                   10


<210>  19
<211>  11
<212>  PRT
<213>  Homo sapiens


<220>
<221>  TSHR-specific autoantibody epitope
<222>  (1)..(11)

<400>  19

Ile Tyr Val Ser Ile Asp Val Thr Leu Gln Gln
1               5                   10


<210>  20
<211>  15
<212>  PRT
<213>  Homo sapiens


<220>
<221>  TSHR-specific autoantibody epitope

<222>  (1)..(15)

<400>  20

Tyr Asn Leu Ser Lys Val Thr His Ile Glu Ile Arg Asn Thr Arg
1               5                   10                  15


<210>  21
<211>  15
<212>  PRT
<213>  Homo sapiens


<220>
<221>  TSHR-specific autoantibody epitope
<222>  (1)..(15)

<400>  21

Thr Lys Val Tyr Ser Thr Asp Ile Phe Glu Ile Leu Glu Ile Thr
1               5                   10                  15


<210>  22
<211>  8
<212>  PRT
<213>  Homo sapiens


<220>
<221>  TSHR-specific autoantibody epitope
<222>  (1)..(8)

<400>  22

Glu Thr Leu Thr Leu Lys Leu Tyr
1               5


<210>  23
<211>  8
<212>  PRT
<213>  Homo sapiens


<220>
<221>  TSHR-specific autoantibody epitope
<222>  (1)..(8)

<400>  23

Leu Tyr Asn Asn Gly Phe Thr Ser
1               5


<210>  24
<211>  8
<212>  PRT
<213>  Homo sapiens


<220>
<221>  TSHR-specific autoantibody epitope

<222>  (1)..(8)

<400>  24

Val Gln Gly Tyr Asp Phe Phe Gly
1               5


<210>  25
<211>  8
<212>  PRT
<213>  Homo sapiens


<220>
<221>  TSHR-specific autoantibody epitope
<222>  (1)..(8)

<400>  25

Gly Thr Lys Leu Asp Ala Val Tyr
1               5


<210>  26
<211>  14
<212>  PRT
<213>  Homo sapiens


<220>
<221>  TSHR-specific autoantibody epitope
<222>  (1)..(14)

<400>  26

Ser Leu Ser Phe Leu His Leu Thr Thr Arg Ala Asp Leu Ser
1               5                   10


<210>  27
<211>  10
<212>  PRT
<213>  Homo sapiens


<220>
<221>  TSHR-specific autoantibody epitope
<222>  (1)..(10)

<400>  27

Phe Phe Glu Glu Gln Glu Asp Glu Ile Leu
1               5                   10